# EUROPEAN PATENT APPLICATION

(11) **EP 2 156 826 A1**
(43) Date of publication of application: **24.02.2010**
(21) Application number: 08019218.0
(22) Date of filing: 27.09.2005
(51) Int. Cl.: A61K 9/50, A61K 31/4422

(54) **Microcapsule formulations comprising two pharmaceutically active ingredients**

(30) Priority: 27.09.2004 US 612784 P; 27.09.2004 US 612785 P; 27.09.2004 US 612786 P
(62) Divisional of application: 05786942.2
(71) Applicant: Sigmoid Pharma Limited, Dublin 9 (IE)
(72) Inventor: Moodley, Joey, Athlone County Westmeath (IE); Coulter, Ivan, Dublin 2 (IE)
(74) Representative: Couchman, Jonathan Hugh

(57) **Abstract**

A formulation comprising a plurality of seamless multiparticulate minicapsules or multiparticulate beads having a diameter of from 0.5 mm to 5 mm, the minicapsules having an encapsulating medium, and the mincapsules containing at least two different active ingredients.

## Description

### Field of the Invention

This invention relates to novel combination approaches to enhance therapeutic benefit or active bioavailability. In some disease treatment instances, more than one active is recommended, either under label or off-label, to increase the overall drug effectiveness or efficacy. In other instances, a combination of actives may enhance the bioavailability of one or all actives.

### Background of the Invention

Drug combination approaches are gaining in popularity. The main drivers are a recognition that such approaches often lead to a better therapeutic outcome and a greater understanding both of the molecular events leading to disease as well as molecular intervention strategies. While a number of combination products are in clinical use, mainly in the cardiovascular field, other desirable combinations have not been possible due to formulation incompatibilities, such as, for example, the fact that lipid-based and water-based formulations do not mix. This invention is directed to formulations which will enable the development of heretofore incompatible drugs being administered as a single pill.

A major issue affecting drug effectiveness is the requirement for high drug dosage resulting in much waste and often toxicities. The underlying reasons necessitating such high dosage include poor drug absorption from the intestine, drug metabolism by enzymes of the cytochrome P450 class, including the various 3A subset family, that reside in the cells lining the intestine and in the liver, drug degradation by low pH in the stomach and enzymes in the small intestine, including protease and peptidases, as well as various cellular pump systems that cause efflux of drugs from the cells, including PgP efflux pumps. Further issues relate to the development of resistance following un-going drug administration. The underlying cause for such resistance often is an inbuilt molecular protective mechanism such as that controlled by histone deacetylase in the case of COPD resistance to corticosteroids.

Co-administering a drug with poor oral bioavailability or stability with metabolism, including cytochrome P450 3A, inhibitors, degradative enzyme inhibitors, efflux inhibitors increase drug bioavailability and stability, thereby increasing drug dosing efficiency, reducing dose size and frequency as well as ensuring that the active drug is less toxic and thus better tolerated.

A further driver for novel drug combinations is that the aetiology of many diseases stems from multiple molecular pathway dysfunction. The result is that while individual drugs, targeting individual pathways, improve the symptoms a number of the underlying causes are left untreated, resulting in a chronic deterioration of the patient's health. Combination therapies, targeting a number of the aetiological malfunctions, have the potential to lead to improved disease management and patient health and wellbeing. Examples of diseases stemming from multiple aetiological are numerous, including cancer, cardiovascular and heart disease, central nervous system disorders, immunological conditions and diabetes.

A major impedance to combination therapy develop stems from the incompatibility of different drug formulations. Generally speaking water soluble drugs are not compatible with lipid- or oil-soluble drugs. Additionally, many labile drugs are effective only for short time periods, often less than 6 hours. As such, patients are required to take multiple pills, often several times a day, a major inconvenience.

Drug formulation approaches are therefore required that address solubility, instability, efficacy, safety, tolerability, convenience and compliance issues.

Drugs that are poorly water soluble must be formulated in a way that improves their solubility and hence their bioavailability. In general, a drug that is in solution or suspension when administered by the oral route is rapidly and frequently instantaneously absorbed from the gastrointestinal tract resulting in a fast therapeutic action. However, in many cases it is desirable to control the rate of absorption following oral administration in order to achieve the desired plasma profile or prolongation of action or to avoid any side effects of the drug.

Numerous processes and formulations exist to enhance the solubility of poorly soluble drug compounds. Solubilisation of such drugs in solvents, oils, emulsions and microemulsions are well known to those skilled in the art and have been used to deliver such drugs orally. Such formulations are then encapsulated in soft gelatine capsules for oral administration. Soft gelatine encapsulation is a specialised process. Soft gelatine capsules do not lend themselves easily to further processing such as the addition of delayed or sustained release coatings.

There is also a need for a formulation which will allow the delivery of a drug to the optimum site of absorption/action in the gastrointestinal tract.

Also, in some cases there is a need for increased time of residence or delivery to certain locations along the GIT, either to optimise systemic bioavailability or localised activity.

Methylxanthines, especially in this case Theophylline, pentoxifylline and A802715 are useful medicines frequently used, due to bronchodilatory effects, as an agent for treating symptoms of bronchial asthma. As known in the art the effective blood levels is about 10-20 mg/L. If the concentration in the blood exceeds 20 mg/L it has been seen to have serious side effects on the cardiovascular and nervous systems (Barnes, PJ. Current Treatments for asthma: Promises and Limitations. Chest 1997; 111:17S-22S). Furthermore difference in blood levels among individuals, cardiac insufficiency, liver and kidney disease, age, smoking etc. also have effects. Recently, it has been suggested that, at lower concentrations of between 5-10 mg/L that Theophylline may have an anti-inflammatory and immunostimulatory effect (Barnes PJ, Pauwels RA. Theophylline in asthma: time for a reappraisal? Eur Respir J 1994; 7: 579-591). Theophylline has a short biological half life <6 hours for adults, therefore it is necessary for multiple dosing (QID) to achieve effective blood levels have been considered. To address this issue of multiple dosing, numerous sustained release type formulations have been developed. However, these sustained release formulation suffer from a number of problems. Principal amongst these is the need for a zero- or pseudo-zero-order release profile in a format that is compatible with other often co-administered drugs, administration of which benefit from sustained release.

Oral administration of corticosteroids is widely prescribed to reduce inflammation in both Asthma and Chronic Obstructive Pulmonary Disorder (COPD) as well as for other inflammation-related diseases. While effective in a majority of asthmatics and a large proportion of COPD patients, long term administration of high corticosteroid doses has been associated with a number of side effects, including osteoporosis, hypertension and weight gain. As a result of COPD-related cellular oxidative stress a large proportion of COPD patients have developed resistance to corticosteroid therapies (Kirsten DK, Wenger RE, Jorres RA, Magnussen H. Effects of theophylline withdrawal in severe chronic obstructive pulmonary disease. Chest 1993; 104: 1101-1107).

To optimise disease management, oral administration of numerous cardiovascular drugs is common place. Various cardiovascular drug combinations are prescribed to treat heart and vascular disease and to prevent initial or further disease related incidences. Additionally, a number of cardiovascular drugs have shown potential and clinical effectiveness in the treatment or prevention of symptoms or events that arise or give rise to other diseases such as diabetes related morbidities and mortality.

A number of interventive strategies to avert an escalating crisis of heart attacks and strokes is ongoing. Gaining prominence as an interventive and prophylactic as well as therapeutic approach is the concept of a single formulation of six medications given to those most at risk. This debate was strengthened by the publication in British Medical Journal (BMJ 2003; 326: 1419 Wald and Law) of a proposal by British medical scientists that all heart patients and all those aged 55 should be targeted to receive a single formulation of six medications, the so-called 'polypill'..

The 'polypill', proposed by Dr Nicolas Wald and Dr Malcolm Law of the University of London, comprises a statin or simvastatin to lower blood LDL-cholesterol, three blood-pressure lowering drugs at half dose (e.g., a thiazide diuretic, a beta-blocker and an angiotensin converting enzyme (ACE) inhibitor, folic acid to reduce levels of homocysteine and subsequent atherosclerosis, and aspirin, as an anticoagulant.

A difficulty associated with development of a single pill containing up to six drug formulations is the incompatibility of the individual drug formulations, incompatibilities such as oil versus water soluble, long versus short half-life, differential circadian-related illness drug chronotherapy requirements. A multicomponent aggregator technology is required to ensure that a single unit 'polypill' is made possible.

In addition to the intended pharmaceutical action, several cardiovascular drug classes have been shown to have a range of activities. These include, for example, Angiotensin II inhibitors attenuate some of the actions that Angiotensin II has been shown to foster, including inflammation, plaque formation, fibrosis and excessive growth of arterial walls and the left ventricle.

As well as Angiotensin II Inhibitors, Animal and clinical studies suggest that ACE inhibitors hinder many of these damaging processes. ACE inhibitors also may benefit people with cardiovascular disease by encouraging new blood vessels to sprout. One ACE inhibitor, quinapril, was recently shown to do this in animal studies. By lessening inflammation and fibrosis, ACE inhibitors are thought to stall the progress of kidney disease. ACE inhibitors can also boost blood flow to the pancreas, reduce insulin clearance from the liver and hamper inflammation.

Additionally, many of the ACE inhibitors are now approved for use in congestive heart failure (The CONSENSUS Trial Study Group. Effects of enalapril on mortality in severe congestive heart failure. Results of the Cooperative North Scandinavian Enalapril Survival Study (CONSENSUS). N Engl J Med 1987;316:1429-35.).

In a range of clinical studies, ACE Inhibitors have been shown to have positive effects on diabetes onset and progression. Following onset of diabetes, however, most studies seem to suggest that ACE inhibitors can help in several ways:
- The drugs cut in half the incidence of cardiovascular events and produce modest reductions in blood pressure.
- ACE inhibitors lower the risk of developing or experiencing a progression of kidney disease by one-quarter, a degree not attainable by other hypertensive drugs-except for angiotensin receptor blockers
- The drugs slow the progression of retinopathy in patients with normal blood pressure levels.

The American Diabetes Association recommends ACE inhibitors for diabetics with microalbuminuria. The organization also recommends the drugs for diabetics over 55 with hypertension, and for diabetics who don't have hypertension but have another risk factor for cardiovascular disease. It is a rare diabetes patient who doesn't meet one of these criteria.

Likewise, statins have been shown to a range of actions such as anti-inflammatory and prevent the growth of Alzheimer's Disease -associated plaque formation (Association between statin use and Alzheimer's disease. Zamrini et al.; Neuroepidemiology. 2004 Jan-Apr; 23(1-2):94-8.).

A recent study, reported in the New England Journal of Medicine (N Engl J Med. 2005 Jul 7;353(1):85-6.), suggests that hydrourea, if combined with a small dose of aspirin is effective in the treatment and prevention of thrombocythemia and is more effective in preventing serious bleeding. Thrombocythemia occurs when too many blood-clotting structures known as platelets are produced in the bone marrow. It can affect other types of blood cells and can cause a host of problems including chest pain, bleeding, and leg and lung clots. It usually strikes in middle age, affecting 1 in 30,000 people. In a Journal editorial, Tiziano Barbui and Guido Finazzi of Ospedali Riuniti in Bergamo, Italy, said "for now, hydroxyurea plus aspirin should be the standard of treatment for patients with essential thrombocythemia" whose disease makes them vulnerable to blood clots.

Even following entry into tumour cells, anticancer agents, often plagued by low solubility and/or poor permability, are susceptible to ejection from the cell through the action of efflux pumps. A number of efflux pump inhibitors has been identified, including cyclosporine A (Efficacy of novel P-glycoprotein inhibitors to increase the oral uptake of paclitaxel in mice. Bardelmeijer et al., Invest New Drugs. 2004 Aug;22(3):219-29.), cimetidine (Impact of gastric acid suppressants on cytochrome P450 3A4 and P-glycoprotein: consequences for FK506 assimilation. Lemahieu et al., Kidney Int. 2005 Mar; 67(3):1152-60.), omerprazole (Interaction of omeprazole, lansoprazole and pantoprazole with P-glycoprotein. Pauli-Magnus et al., Naunyn Schmiedebergs Arch Pharmacol. 2001 Dec;364(6):551-7.), Vitamin E TPGS (Enhanced oral paclitaxel absorption with vitamin E-TPGS: effect on solubility and permeability in vitro, in situ and in vivo. Varma MV, Panchagnula R; Eur J Pharm Sci. 2005 Jul-Aug;25(4-5):445-53.), verapimil (Verapamil metabolites: potential P-glycoprotein-mediated multidrug resistance reversal agents. Woodland et al., Can J Physiol Pharmacol. 2003 Aug;81 (8):800-5) as well as metabolites or therapeutically inactive enantiomers.

To optimise bioavailability and therapeutic benefit, in addition to combining poorly permeable drugs with permeability enhancers or solubility enhancers, there is a need to further add efflux pump inhibitors (Coadministration of Oral Cyclosporin A Enables Oral Therapy with Paclitaxel, Meerum Terwogt; Clinical Cancer Research Vol. 5, 3379-3384, November 1999). A further issue with cancer treatment is inherent immunosuppression and red blood cell death associated with several anticancer agents. Consequently, a significant side effect in anti-cancer treatment relates to immunosuppression or immunoablation wherein cells in the immune system are destroyed thereby reducing the capability of the immune system to protect the body from opportunistic infections. To prevent or overcome immunosuppression may require the co-administration of immunostimulators such as inosine or blood enriching agents such as erythropoietin. A number of approaches demonstrating the effectiveness of co-administering immunostimulatory, immunomodulatory and cytoprotecting agents with anti-cancer have been proposed and tested (Immunomodulation with interferon-gamma and colony-stimulating factors for refractory fungal infections in patients with leukemia. Dignani et al., Cancer. 2005 Jul 1;104(1):199-204; Cytoprotection and immunomodulation in cancer therapy. Diwanay et al., Curr Med Chem Anti-Canc Agents. 2004 Nov;4(6):479-90.).

Treatment of infectious diseases such as HIV/AIDS, Malaria and Tuberculosis often requires the administration of drug cocktails to effectively control the viral or bacterial titres to manageable levels. As with other cocktails, the problem associated with combinations is differential inherent drug solubility, permeability and stability profiles. Additionally, some effective drugs are susceptible to efflux pumps. Therefore, effective treatments will require the development of formats capable of bundling previously incompatible drug formulation types that improve low solubility, enhance permeability and control release of unstable drugs with short half lives. Two further issues relate to efflux and immunosuppression. Therefore, the addition of efflux inhibitors and/or immunostimulators to cocktails may improve bioavailability and the overall effectiveness of treatment.

The number of biopharmaceutical drugs in development and entering the clinic is growing at a rate double that of conventional small molecules. Most peptide and protein drugs are currently used as parenteral formulations because of their poor oral bioavailability. Development of an effective oral delivery system for these macromolecular drugs requires a thorough understanding of their physicochemical properties, such as molecular weight, hydrophobicity, ionization constants, and pH stability, as well as biological barriers that restrict protein and peptide absorption from the gastrointestinal (GI) tract, including pH variability, enzymatic degradation, and membrane efflux. Various strategies currently under investigation include amino acid backbone modifications, formulation approaches, chemical conjugation of hydrophobic or targeting ligand, and use of enzyme inhibitors, mucoadhesive polymers, and absorption enhancers. However, there is only limited success because of the hostile environment of the GI tract, including strong pH extremes and abundant presence of potent luminal enzymes (Emerging Trends in Oral Delivery of Peptide and Protein Drugs, Mahato et al., Critical Reviews™ in Therapeutic Drug Carrier Systems, Issues 2 & 3, 2003).

It has long been acknowledged that for vaccines to be effective requires the co-administration of antigenic materials with adjuvants which stimulate the immune system to identify and illicit a immunological response to the immunogenic antigen. For decades vaccines depended on adjuvants such as Alum and Freund's. A limitation with the above adjuvants is that synergies do not exist between all vaccines. Recently, a broad range of adjuvants has been developed that have demonstrated varying activity depending on the immunogenic antigen being administered. Adjuvants formulated as water in oil microemulsions are replacing the more toxic aluminum hydroxide formulations for veterinary use. These new adjuvants allow for products that are highly effective with minimal adverse reactions. One example is the development of MF59 as an adjuvant (Estuningsih SE, Smooker PM, Wiedosari E, et al. Evaluation of antigens of Fascioloa gigantica as vaccines against tropical fasciolosis in cattle. Int J Parasitol. 1997;27:1419-1428.).

A further lipid-based adjuvant class are the saponins, a heterogeneous group of sterol glycosides and triterpene glycosides which are common constituents of plants. One source of triterpenoid saponins obtained from the bark of Quillaia saponaria Molina (the soap bark tree) have been known to cause substantial enhancement of immune responses since the 1920s. Despite their use in animal vaccines, the development of saponin-based formulations for human vaccines has been impeded by their complexity and concerns about toxicity. Recently, the improved molecular understanding of the relationships between adjuvant activity, toxicity and structure of saponins and formulation of saponins into structures with reduced toxicity such as ISCOMs has led to testing in humans (ISCOMs and other saponin based adjuvants, Barr et al., Advanced Drug Delivery Reviews; Vol. 32, No. 3, pages 247-271, 1998).

A related issue pertains to oral vaccination. Despite the intestinal mucosa being rich in immune cells such as M-cells and Peyer's patches, with the exception of certain live or attenuated live vaccines such as that for Polio, oral vaccination has disappointed (Cholera toxin B subunit conjugated bile salt stabilized vesicles (bilosomes) for oral immunization. Singh et al., Int J Pharm. 2004 Jul 8;278(2):379-90; Exploiting receptor biology for oral vaccination with biodegradable particulates. Foster and Hirst, Adv Drug Deliv Rev. 2005 Jan 10;57(3):431-50; Mice fed lipid-encapsulated Mycobacterium bovis BCG are protected against aerosol challenge with Mycobacterium tuberculosis. Adwell et al., Infect Immun. 2005 Mar;73(3):1903-5). To improve the oral effectiveness will require the local release of antigens and associated adjuvants proximal to the immune cells.

Abuse of prescription drugs is a growing phenomenon. Anti-depressants, sleep-inducing agents, amphetamines and painkillers are widely abused. OxyContin, a powerful and addictive painkiller, which provides a heroin-like high, and Ritalin, a stimulant used to treat attention deficit hyperactivity disorder, are among often-misused prescription drugs. Commonly, such drugs are transformed from a pill or capsule form and injected intravenously. To counteract abuse, a number of approaches has been developed, most of which involve the addition of a noxious irritant or, alternatively, an antidote to the active ingredient with the pill or capsule excipients. When injected, the antidote inactivates the active ingredient whereas the irritant causes a burning or other unpleasant sensation at the site of action. Improving tamper proofing will require novel controlled release combination products, enabling release of the active in the small intestine for maximal absorption and release of the antidote or irritant in the colon where absorption of the active is minimal. Selected antidotes or irritants will be innocuous when released in the colon and generally will not be absorbed.

To counteract the growing antibiotic resistance problem a number of approaches is being adopted to enable delivery of the antibiotic while preventing damage to the intestinal, mainly colonic, bacterial flora. One such approach is a novel antibiotic-enzyme combination that involves co-administering an antibiotic with an enzyme which degrades and thus inactivates the antibiotic. This approach requires a delivery technology that will release the antibiotic in the upper small intestine and the enzyme at the junction of the small and large intestine.

The invention relates to various drug delivery formats that permit the development of combination therapies in a single pill format and enables once-daily administration through using various controlled release technologies and tablet formats.

A major obstacle to the development of effective and user-friendly combination pills are significant drug and formulation incompatibilities. Incompatibilities include water versus oil solubility, short versus long half-lives, food versus fasted dosing and different intestinal sites of action or absorption. This invention seeks to address these issues.

### Summary of Invention

According to the invention there is provided a pharmaceutical formulation comprising a plurality of seamless minicapsules having a diameter of from 0.5 mm to 5 mm, at least some of the minicapsules containing a methyxanthine as one active ingredient, and at least some of the minicapsules containing a corticosteriod as another active ingredient.

In one embodiment at least some of the minicapsules contain both a methyxanthine and a corticosteroid.

The minicapsules may comprise an encapsulating medium. The encapsulating medium of at least some of the minicapsules may contain at least one of the active ingredients.

In one embodiment at least some of the minicapsules comprise a core. The core may contain at least one active ingredient.

In one embodiment at least some of the minicapsules comprise at least one layer. The layer may contain at least one active ingredient. The layer may comprise a coating to control the time and/or location of the release of the active entity.

The coated seamless minicapsules may have a diameter of from 0.5 mm to 5.0 mm, from 1.2 mm to 2.0 mm, 1.4 mm to 1.8 mm.

In one embodiment at least one coating is an immediate release coating, a sustained release coating, a sustained release and an immediate release coating, an enteric coating, or a bioadhesive coating such as a mucoadhesive coating.

In one embodiment the layer comprises a buffer layer.

In one embodiment the minicapsule is formed from a core solution containing an active ingredient, and an encapsulating solution which forms, on setting, the encapsulating medium. The encapsulating solution may contain an active ingredient. The active ingredient contained in the encapsulating solution may be the same as or different from the active ingredient in the core solution. The active ingredient contained in the encapsulating solution may be in a micronised or nanonized particle form. The minicapsule may be formed from a solution containing the encapsulating medium and an active ingredient.

In one embodiment the formulation comprises at least two different populations of minicapsules. One population of minicapsules may contain a methyxanthine and another population of minicapsules contains a corticosteroid.

In one embodiment about 50% of the formulation is comprised of a population of sustained release seamless minicapsules of a methylxanthine and a population of about 50% of a sustained release seamless minicapsule of a Corticosteroid. The ratio of seamless minicapsules of a Methylxanthine to a Corticosteroid may be about: 95:5, 90:10, 80:20, 70:30, 60:40, 50:50, 5:95, 10:90, 20:80, 30:70 or 40:60.

The invention also provides a formulation comprising a methylxanthine and a corticosteroid in a multiparticulate seamless oral formulation. The formulation may comprise sustained release particles having a core containing a methylxanthine and/or a corticosteroid in a pharmaceutically acceptable solvent or liquid phase encapsulated into multiparticulate seamless minicapsules. Alternatively the formulation may comprise sustained release particles having a core containing a methylxanthine and/or a corticosteroid in a pharmaceutically acceptable solvent or liquid phase encapsulated into multiparticulate seamless minicapsules encapsulated with a gelatine shell containing a methlyxanthine and/or a corticosteroid. The seamless minicapsules may be coated with a rate-controlling or enteric coating to achieve effective plasma levels of the methylxanthine and the corticosteroid over a period of at least 12 or 24 hours. An immediate release coating may be provided in addition to the rate-controlling coating. The immediate release coating may contain a methylxanthine. Alternatively or additionally the immediate release coating contains a corticosteroid.

In one embodiment the immediate release formulation comprises seamless minicapsules containing a core of a methylxanthine and/or a corticosteroid solubilised or suspended or dispersed in a liquid phase.

In one case the formulation comprises at least two populations of sustained release minicapsules having different in-vitro dissolution profiles. The formulation may provide a dissolution profile in a pre-determined media such that about 0-25% of the combined product is released after 1 hour, about >25% released after 3 hours, about >50% released after 6 hours and >80% released after 12 hours. Alternatively the formulation may provide a dissolution profile in a pre-determined media such that <15% of the combined product is released after 1 hour, about >15% is released after 3 hours, about >35% is released after 9 hours, about >45% is released after 12 hours and >80% is released after 24 hours.

The methylxanthine may be selected from theophylline, pentoxifylline, and A802715.

The corticosteroid may be selected from dexamethasone, prednisolone, prednisone and budesonide.

The invention also provides a capsule containing a plurality of minicapsules of the invention. The capsule may contain another entity.

In another aspect the invention provides a formulation comprising a plurality of seamless multiparticulate minicapsules having a diameter of from 0.5 mm to 5 mm, the minicapsules having an encapsulating medium, and the mincapsules containing at least two different active ingredients. The encapsulating medium of at least some of the minicapsules may contain at least one of the active ingredients. At least some of the minicapsules may comprise a core. The core may contain at least one active ingredient.

At least some of the minicapsules may comprise at least one layer. The layer may contain at least one active ingredient. The layer may comprise a coating to control the time and/or location of the release of the active entity.

The coated seamless minicapsules may have a diameter of from 0.5 mm to 5.0mm, from 1.2 mm to 3.0 mm, from 1.4 mm to 1.8 mm.

At least one coating may be an immediate release coating, a sustained release coating, a sustained release and an immediate release coating, an enteric coating, or a bioadhesive coating such as a mucoadhesive coating.

In one embodiment the layer comprises a buffer layer.

In one case the minicapsule is formed from a core solution containing an active ingredient, and an encapsulating solution which forms, on setting, the encapsulating medium. The encapsulating solution may contain an active ingredient. The active ingredient contained in the encapsulating solution may be the same as or different from the active ingredient in the core solution. The active ingredient contained in the encapsulating solution may be in a micronised or nanonized particle form.

In one embodiment at least some of the minicapsules are formed form a solution containing the encapsulating medium and an active ingredient.

The formulation may comprise at least two different populations of minicapsules. One population of minicapsules may contain a first active ingredient and another population of minicapsules may contain a second active ingredient.

The formulation may comprise a capsule containing a plurality of minicapsules. The capsule may contain another entity. The other entity may be in a liquid, powder, solid, semi-solid or gaseous form. The other entity may comprise an active entity.

In another embodiment the formulation comprises a tablet or pellet containing a plurality of minicapsules. The tablet or pellet may contain another entity. The other entity may be an active entity.

The formulation may be:-
a combination product comprising a methylxanthine and an anticancer agent (such as cisplatin, paclitaxel, daubomycin or vincristine);
a combination product comprising a methylxanthine and a Vitamin A analogue (such as valproaic acid, valproate or isotretinoin);
a combination product comprising a methylxanthine and a nitric oxide donor such as nitroprusside, 02-acyl diazenium diolole or NO-NSAIDs such as NO-aspirin;
a combination product comprising a methylxanthine and a reactive oxygen species scavenger such as stephenhenanthrine or uvariopsine;
a combination product comprising an immunostimatory agent such as inosine or other adjuvants and an anticancer agent such as cisplatin, paclitaxel, daubomycin or vincristine;
a combination product comprising various antiretroviral agents for the treatment of HIV/AIDS, selected from sequinivir, stavudine, ritonivir, lipinavir, amprenevir;
a combination product comprising various antiretroviral agents for the treatment of HIV/AIDS together with immunostimulatory agents;
a combination product for the treatment of malaria comprising Artemisinin-based actives, including artesunate plus sulfadoxine/pyrimethamine or artesunate and amodiaquine;
a combination product for the treatment of tuberculosis comprising isoniazid, rifampin and pyrazinamide;
a combination product for the co-treatment of HIV/AIDS, Malaria and TB, comprised of, from one of the following: HIV: Sequinivir, Stavudine, Ritonivir, Lipinavir, or Amprenevir; Malaria: Sulfadoxine/Primethamine/Artesunate; and Tuberculosis: Isoniazid/Rifampin/Pyrazinamide;.
a combination product comprising various cardiovascular agents, selected from one or more of ACE inhibitors, antidiuretics, statins, anticholesterol agents, anti-coagulants, beta-blockers and anti-oxidants;
a combination product comprising immunomodulators including vaccines and immunotherapeutic agents with immunostimulatory and/or adjuvants;
a combination product comprising a proton pump inhibitor (PPI) [which may be selected from omeprazole, lansoprazole, rabeprazole, esomeprazole, pantoprazole], an anti-H-Pylori antibiotic [which may be selected from metronidazole, tetracycline, clarithromycin, amoxicillin], H-blockers [which may be selected from cimetidine, ranitidine, famotidine, nizatidine] and stomach lining protectants [such as bismuth subsalicylate], the PPI and H-blockers being released following transit through the stomach, the antibiotic release in the stomach and the stomach lining protectant being released in the stomach;
a combination product comprising agents susceptible to efflux pump activity or metabolism via cytochrome P450 subtypes, including 3A, together with inhibitors of such;
a combination product comprising an antibiotic susceptible to enzymatic degradation and a degradative enzyme, the antibiotic have a controlled release profile in the stomach and small intestine and the enzyme being released in the distal small intestine and colon;
a combination product comprising a narcotic, anti-psychotic or other potentially addictive agent with an antidote or irritant, the former drug classes being released in the stomach and small intestine with the antidote, an innocuous or non-systemically absorbed agent, being released in the colon, the irritant may be irritating when injected but innocuous when taken orally;
a combination product for the treatment of Alzheimer's Disease comprising a cholinesterase inhibitor (such as donepezil, rivastigmine, galantamine) and a N-Methyl-D-Aspartame (NMDA) antagonist such as memantine;
a combination product for the treatment of Alzheimer's Disease comprising a cholinesterase inhibitor (such as donepezil, rivastigmine, galantamine) and one or more from the following classes: vitamins, statins, estrogen, nootrophic agents, ginkgo biloba, anti-inflammatory agents, anti-depressants, anti-psychotics, mood stabilizers and calcium channel blockers, including Nimodipine;
a cholesterol lowering combination product comprised of a HMG-CoA inhibitor and a intestinal cholesterol uptake inhibitor;
a combination product for the treatment of diabetes comprising insulin and an insulin sensitizer;
a combination product for the treatment of diabetes comprising insulin and an oral antihyperglycemic agent;
a combination product for the treatment of diabetes comprising insulin and a sulfonylurea agent or metformin;
a combination product for the treatment of diabetes comprising insulin and an oral PTP-1B inhibitor;
a combination product for the treatment of diabetes comprise an oral memetic agent with an appetite suppressant or fat uptake inhibitor such as orlistat;
a combination product comprising an anti-cancer agents and a potency enhancers, including isoflavanoids, polyphenols and anti-cancer agent derivatives;
a combination product containing a potency enhancer such as an isoflavanoid and either a heart disease therapy, osteoporosis therapy, autoimmune disease treatment or inflammatory bowel disease treatment.

In one embodiment of the various aspects of the invention at least some of the minicapsules are provided with a bioadhesive such as a mucoadhesive.

The bioadhesive may comprise from 0% to 10% by weight of one or more of the following polymer classes:- polyacrylates; polyanhydrides; chitosans; carbopols; cellulose; methylcellulose; methylated deoxycellulose (m-doc^{™)}, lectins.

The bioadhesive may comprise from 0% to 10% by weight of one or more of the following thiolated or otherwise derivated polymers:- ppolyacrylates; polyanhydrides; chitosans; carbopols; cellulose; methylcellulose; methylated deoxycellulose (m-doc^{™)}, lectins.

The bioadhesive may comprise a coating. Alternatively or additioanlly the bioadhesive is incorporated into a part or layer of the minicapsule such as into the rate-controlling layer and/or into the encapsulating medium.

In another embodiment at least some of the minicapsules have have a layer such as an outer layer which is divided into a least two parts. The parts may be of the same or different compositions.

The formulation may comprise hard gelatine capsules with solid, semisolid or liquid cores.

The formulation may comprise a sachet, a sprinkle, a suppository for anal or vaginal or intrauterine delivery, a suppository or sprinkle for buccal delivery, nasal delivery or pulmonary delivery.

The present invention, seeks, through bundling in an innovative way, several approaches as novel combinations. Starting with poor solubility, adding controlled release and muco- or bio-adhesive polymers, and including more than one active or an active and an adjuvant, the current approach addresses several unmet and challenging needs.

The invention provides an oral formulation or process which can be used to administer the solubilised and/or dispersion of the active ingredient as a combination product in a manner which allows the formulation to be subsequently coated to deliver the active at a predetermined site of absorption and/or at a predetermined rate of delivery consistent with the optimum absorption and bioavailability or plasma profile of the drug.

The invention also provides an oral formulation which can be used to administer one or more active ingredient of differing solubility which, released in a predetermined manner to target sites in the gastrointestinal tract, achieve maximum absorption at the site of release of the active ingredient.

The oral formulation of the invention can provide the release of an active ingredient in the gastrointestinal tract in a manner which minimises high local concentrations of solid active ingredient, thereby reducing intestinal lining irritation. Multiparticulate drug delivery systems by their nature allow the release of the active ingredient over a larger surface area of the gastrointestinal tract thereby minimising high localised drug concentration for drugs which are irritants to the gastrointestinal tract, thus reducing any associated side effects.

This invention permits the development of broad and novel combination therapies, combining previously incompatible formulations, whether:
- water or oil soluble,
- in powder or liquid/emulsion form,
- in liquid, semi-liquid or solid form
- exhibiting different half lives,
- small molecule or biopharmaceutical,
- therapeutic or adjuvant,
- released in stomach, small or large intestine.

The minicapsule core in this invention may be comprised of a solid, a semi-solid or a liquid core. Furthermore, the minicapsules may be coated with one or several coating combinations. Coatings may contain one or more of the following:
- Active drug
- Adjuvants
- Bio- or mucoadhesive agents
- Controlled release polymers
- Taste-masking entities
- Moisture prevention or retention entities
- Oxidation prevention or retention
- Light blocking agents

To enable delivery of the complex anti-cancer drug combinations cited above requires the development of a sophisticated drug delivery aggregator or bundling technology. The present invention enables the bundling or aggregation of complex drug combinations.

The formulations of the invention permit technology bundling. The potential to formulate non-conjugated or conjugated biopharmaceuticals with or without permeability enhancing excipients, encapsulate such into minicapsules or solid minispheres and then coat with muco- or bio-adhesives and/or controlled release polymers is enabled by the current invention.

The invention provides a formulation with an antigen and an adjuvant in a minicapsule core, buffer or shell, dual coating the minicapsule with an inner mucoadhesive coat and an outer controlled release coating. The minicapsules will adhere to regions rich in M-cells and Peyer's Patches and release the adjuvant and antigen in sufficient concentrations locally to ensure a strong immune response.

The invention facilitates combinations to be developed so that prospective addicts will be unable to distinguish between capsules containing the active from those containing an antidote or irritant and thus suffer the consequences or abstain from abusing the drug.

The invention also provides a formulation for co-administration colonically of 'good' bacteria with small intestinal antibiotic release.

The invention includes the following drug delivery/tablet formats:
- A bead format whereby concentric layers of drug are coated onto an inert bead
- A bead format whereby the core is comprised of an active drug that is further coated with concentric layers of active drug
- A hard gelatine capsules containing controlled release beads
- A hard gelatine capsule containing individual drugs in mini-pellet form
- A compressed tablet wherein individual drugs are separated by means of layering
- A compressed tablet wherein individual controlled release drug beads are compressed into a tablet form

The invention permits the development of various combinations, enabling different drug release profiles, in various tablet or pill formats. The invention caters for various administrative formats such as drugs that are readily available in free-flowing powder form, and/or drugs that are optimally formulated in liquid/emulsion form, such as those exhibiting poorly soluble or poorly permeable physicochemical properties. Thus the present invention overcomes and caters for all drug formulations, including previously incompatible formulations.

Combination Therapy Advantages:
- Simpler - improved compliance and treatment outcome
- Reduce error in mediacation
- Reduce resistance
- Synergistic combinations
   o Improved disease management
   o Enhanced bioavailability
   o Safety
- Reduced shortages (logistics)
- One expiry date
- Easier procurement, management and handling
- Reduced production, packaging and shipping costs
- Reduced side effects
- Improved tamper-proofing - reduced abuse

In particular, the invention provides an oral Methylxanthine, most especially Theophylline, pentoxifylline (POF) or A802715 as a multiparticulate seamless minicapsule formulation in combination with a Corticosteroid most especially Dexamethasone, Prednisolone, Prednisone or Budesonide for twice (bid) or once daily (qd) administration to a patient, comprising sustained release particles each having a core containing Theophylline or one of the above named corticosteroids in a pharmaceutically acceptable solvent or liquid phase and encapsulated into multiparticulate seamless minicapsules in a range of 1-5 mm in diameter. The encapsulated Theophylline/Corticosteroid minicapsules are coated with a rate-controlling polymer coat comprised of amino methacrylate copolymers in an amount sufficient to achieve therapeutically effective plasma levels of the above combined medicaments over at least 12 or 24 hours in the patient with said condition Asthma/COPD.

The product can also be formulated as an immediate release (IR) dosage form which has an Immediate Onset of Action to provide sufficient relief to the patient within a relatively short period.

The product may also be formulated to provide increased residence time in specific areas of the GIT, maximising release either in the stomach, small intestine or the colon. Controlling the release profile can have a chronotherapeutic effect, especially important in the control of nocturnal breakthrough diseases, including asthma or COPD events. Also, localised release can enhance activity of individual or combination therapies in other inflammatory conditions such as inflammatory bowel disease and Crohn's Disease.

This invention provides a product to address the issue of multiple dosing of a Methylxanthine, such as low dose (5-10 mg/L) Theophylline with a corticosteroid. The combination product should serve to increase the responsiveness of COPD, asthma and other inflammatory disease patients to a corticosteroid. The complementary actions of each drug will reduce side effects associated with the individual higher doses required for activity when either is administered alone.

The invention provides an oral combination product of a Methylxanthine, preferably either of Theophylline, pentoxifylline and A802715 and a Corticosteroid either of Dexamethasone or Prednisolone, Prednisone or Budesonide as a multiparticulate seamless minicapsule formulation for twice or once daily administration to a patient, comprising sustained release particles each having a core containing the active ingredient in a pharmaceutically acceptable solvent or liquid phase and encapsulated into seamless multiparticulate seamless minicapsules in a range of 0.50 - 5.00mum in diameter, more especially in the range 1.50 - 2.00 mm. The combination product of Methylxanthine and Corticosteroid seamless minicapsules may be coated with a rate-controlling polymer coat comprised of amino methacrylate copolymers or an Enteric Coat of a similar amino methacrylate but a different grade in an amount sufficient to achieve therapeutically effective plasma levels of the combined active drugs over at least 12 or 24 hours in the patient.

Additionally, the minicapsules may be coated with at least one or more of the following coatings:
- at least one nitrogen containing polymer, comprising of at least one polyacrylate and or/one poly-N-vinylamide and/or one poly-N-vinyl-lactame, polyacrylamide and/or polyvinylpyrrolidone being preferred
- at least one sulphur containing or thiolated polymer coating, comprising of at least one thiolated cellulose or ethylcellulose derivative and/or a thiolated polyacrylate and/or a thiolated polyacrylamide and/or a thiolated polyvinylpyrrolidone
- at least one polymer coating, comprising of at least one cellulose, ethylcellulose or cellulose analogue, chitosan or chitosan analogues being preferred

The minicapsules have a diameter in the 0.5 to 5.0 mm range, preferably in the 0.5-3.0 mm range, preferably between 1.2-2.0 mm and more preferably in the 1.4-1.8mm range

The minicapsules are capable of extended residence times in the small intestine for a period of at least 5 hours, preferably at least 7 hours and more preferably in the 8-24 hour range to enable maximal bioactivity of the core active agent, locally or systemically

The minicapsules are capable of extended residence times in the large intestine for a period of at least 5 hours, preferably at least 7 hours and more preferably in the 8-24 hour range to enable maximal bioactivity of the core active agent, locally or systemically

The minicapsules are capable of extended residence times in the gastric environment for a period of at least 5 hours, preferably at least 7 hours and more preferably in the 8-24 hour range to enable maximal bioactivity of the core active agent, locally or systemically

In one embodiment, a portion or all of the sustained release minicapsules are further coated with an immediate release a Methylxanthine, one of theophylline, pentoxifylline or A802715 or a Corticosteroid, one of dexamethasone, prednisolone, predisone or Budesonide solution onto the rate-controlling polymer coat.

In an alternative embodiment, the formulation can contain a portion of immediate release seamless minicapsules each comprising a core of a methylxanthine, e.g. theophylline, pentoxifylline or A802715 or a Corticosteroid either of the following dexamethasone, prednisolone, prednisone or Budesonide solubilised or suspended or dispersed in a liquid phase.

In one embodiment the formulation comprises at least two populations of sustained release minicapsules having different in-vitro dissolution profiles.

In one embodiment the formulation provides a dissolution profile in a pre-determined media such that about 0-25% of the combined product is released after 1 hour, about >25% released after 3 hours, about >50% released after 6 hours and >80% released after 12 hours.

In an alternative embodiment, the formulation provides a dissolution profile in a pre-determined media such that <15% of the combined product is released after 1 hour, about >15% is released after 3 hours, about >35% is released after 9 hours, about >45% is released after 12 hours and >80% is released after 24 hours.

In one case, greater than 80% of the formulation is comprised of sustained release minicapsules.

In a preferred embodiment 50% of the formulation is comprised of a population of sustained release seamless minicapsules of a methylxanthine of either theophylline, pentoxifylline or A802715 and a population of 50% of a sustained release seamless minicapsule of a Corticosteroid, in this case either Dexamethasone or Prednisolone or Prednisone or Budesonide.

In a preferred embodiment, the ratio of seamless minicapsules of a Methylxanthine to a Corticosteroid can be formulated according to the following formulae: - 95:5/90:10/80:20/70:30/60:40/50:50/5:95/10:90/20:80/30:70/40:60

The rate-controlling polymer coat may contain Ammonio Methacrylate Copolymer as described in USP /NF in the following ratio's 5:95; 10:90;15:85 as a mixture of Eudragit RL:Eudragit RS more especially Eudragit RL 12.5:Eudragit RS 12.5

The Enteric Coating Polymer may be Eudragit S 12.5 or Eudragit S 30D

The rate-controlling polymer coat may be of Eudragit S 12.5 providing 0 drug release in the stomach for up to 2-6 hours.

The invention also provides a formulation in which a percentage of the enteric coated Corticosteroid seamless minicapsules (Example 3) and a percentage of the coated Methylxanthine seamless minicapsules (Example 2) were blended as per in Example 1.The said blended components were filled into suitable hard gelatin capsules to the required target dosage strength.

In one embodiment the Methylxanthine seamless minicapsules, of either theophylline, pentoxifylline or A802715 as per Example 2 is filled into hard gelatin capsules as a single component to the required target dosage for the products specified indication, excluding the Corticosteroid component.

In another embodiment the Corticosteroid seamless minicapsules in this case either Dexamethasone or Prednisolone or Prednisone or Budesonide as per Example 3 is filled into hard gelatin capsules as a single component to the required target dosage for the products specified indication, excluding the Methylxanthine of either theophylline, pentoxifylline or A802715

In one case, the combination minicapsule formulations may have an increased residence time and release the actives locally in the small intestine

The combination minicapsule formulations may have an increased residence time and release the actives locally in the colon

The combination minicapsule formulations may have an increased residence time and release the actives locally in the stomach

The coatings may be modulated to permit time specific release, thereby enabling chronotherapies for circadian related diseases, especially nocturnal breakout disease conditions and cardiovascular conditions.

In one embodiment a methylxanthine is combined with other immunostimulatory or immunomodulatory actives, including for example cyclosporine.

In other embodiments a methylxanthine is combined with either of one or a combination of the following actives, including anticancer agents, proapoptotic agents, Vitamin A analogues, Nitric Oxide Donors or Reactive Oxygen Species Scavengers.

More generally, the invention provides an oral multiple minicapsule-based product, each minicapsule comprising at least one layer, the core of each minicapsule comprising water-soluble, water-insoluble or partially water-soluble actives in liquid or solid form, a buffer layer comprise of a suitable oil, coated with one or several coats comprised rate-releasing, mucoadhesive or bioadhesive coatings alone or blended, in any combination thereof.

The liquid may be selected from the group consisting of a solution, a spirit, an elixir, a spray, a syrup, an emulsion, a microemulsion, a nanoemulsion, a nanosuspension, a wax, an aerosol, a gel, a foam, a solid foam and a fluid extract.

The shell or solid core may be formed of a material selected from the group consisting of gelatin, starch, casein, chitosan, soya bean protein, safflower protein, alginates, gellan gum, carrageenan, xanthan gum, phtalated gelatin, succinated gelatin, cellulosephtalate-acetate, oleoresin, polyvinylacetate, hydroxypropyl methyl cellulose, polymerisates of acrylic or methacrylic esters, polyvinylacetate-phtalate and combinations thereof.

The minicapsules may be coated with at least one coating comprising at least one nitrogen-containing polymer and/or sulphur-containing or thiolated polymer and/or cellulose or cellulose polymer coating.

The nitrogen-containing coating may be selected from one or more of:
a polyacrylate; a poly-N-vinylamide; and a poly-N-vinyl-lactame.

The nitrogen-containing polymer may be a polyacrylamide or polyvinylpyrollidone.

The cellulose or cellulose derived polymer may be selected from any one or more of:
an ethylcellulose or derivative
a methylcellulose or derivative
a chitosan or derivative.

The mucoadhesive or bioadhesive is selected from any one or more of: Polyacrylates; Polyanhydrides; Lectins; Chitosan; Chitosan glutamate; Polycarbophil; and Carbopol™

The sulphur-containing polymer may be selected from any one or more of: a thiolated cellulose or ethylcellulose derivative; a thiolated polyacrylate; a thiolated polyacylamide; and a thiolated polyvinylpyrollidone.

The polymer coating may be one or more controlled release, mucoadhesive or bioadhesive polymer in any combination or blend.

The multiparticulate capsules may include an antimicrobial selected from the group consisting of paraben and sorbic acid.

The ingredient introduced in said primary capsule comprises a moisture content in the range of about 0% to 6% by weight, generally about 0% to 3% by weight.

Primary and secondary capsules may contain at least one pharmaceutically acceptable lubricant in the range of about 0% to 10% by weight. The lubricant may be selected from the group consisting of aluminiumstearate, calciumstearate, magnesiumstearate, tinstearate, talc, sodium lauryl sulfate, lecithins, mineral oils, stearic acid, silicones and combinations thereof.

In one embodiments the coatings are modulated to permit time specific release, thereby enabling chronotherapies for circadian related diseases, including, but not limited to, nocturnal breakout disease, cardiovascular disease, asthma, respiratory conditions, CNS, autoimmune diseases such as rheumatoid arthritis and oesteoarthritis.

The coatings may be modulated to permit in addition to specific time release modified transit times in the oesophagus, gastric, small intestine, colon or rectum, through the inclusion of muco- or bio-adhesives in the gelatine shell or coated, alone or in combination with controlled release polymers, onto the gelatine shell.

The formulations may comprise another active ingredient such as other immunostimulatory or immunomodulatory actives, including for example cyclosporine, tacrolimus, sacrolimus, inosine or derivates thereof.

The formulation may be encapsulated into hard gelatin capsules, filled into a sachet, used as a sprinkle or as a suppository.

These and other features are included in the claims which are incorporated therein.

### Detailed Description

The invention provides oral Methylxanthines, most especially in this case Theophylline, pentoxifylline or A802715 multiparticulate seamless minicapsule formulation in combination with corticosteroids, most especially Dexamethasone, Prednisolone, Prednisone or Budesonide for twice or once daily administration to a patient. The product comprises sustained release particles each having a core containing one or both of the active ingredients in a solvent or liquid phase as a seamless minicapsule, the core being coated with a rate-controlling polymer coat comprised of ammonia methacrylate copolymers in an amount sufficient to achieve therapeutically effective plasma levels of the combination product over at least 12 or 24 hours. In another embodiment, the core may contain an active ingredient, for example a corticosteroids, in a solvent or liquid phase with another drug, for example a methylxanthine embedded in the gelatine shell, blended with the various shell coatings or included with the intermediate buffering oil layer. Additionally, the coating may contain mucoadhesives which increase the residence time at particular GIT locations. Such mucoadhesives may be applied together with rate-releasing polymers or added as an individual coat, over or under the rate-releasing coat.

In a further embodiment, the core is comprised of a solid, formed from molten gelatine or gelatine-like seamless spherical particles, these particles being coated with rate-limiting polymer coatings or muco-/bio-adhesives in any blend or number of alternating coatings. Yet another embodiment is composed of a solid or semisolid (waxy) material that is liquid at process temperature but solid or semi-solid at room temperature, these particles being coated with rate-limiting polymer coatings or muco-/bio-adhesives in any blend or number of alternating coatings.

The Methylxanthine and the Corticosteroid seamless minicapsules were manufactured according to Freund Industrial Co, Ltd. US Patent No 5,882,680 (Seamless Capsule and Method of Manufacturing the Same), the entire contents of which are incorporated herein by reference.

The principle of seamless minicapsule formation is the utilisation of "surface tension", when two different solutions (which are not or hardly dissolved with each other) contact each other, which works by reducing the contact area of the two different solutions.

After encapsulating the core solution which is ejected through an orifice with a certain diameter, with the shell solution which is also ejected through an outer orifice, the encapsulated sphere is then ejected into a cooling or hardening solution and the outer shell solution is gelled or solidified. This brief describes the formation of seamless minicapsules.

The core solution is mainly a hydrophobic solution or suspension. The outer shell solution is normally gelatin based. However a hydrophilic solution can also be encapsulated with the existence of an intermediate solution, which can avoid the direct contact of the hydrophilic core solution with the outer shell.

With the nozzle having a single orifice, a minicapsule or a bead of shell/core mixed suspension can be processed.

With the nozzle having two orifices (centre and outer),a hydrophobic solution can be encapsulated.

With the nozzle having three or more orifices seamless minicapsules for various applications can be processed. (Ref US Patent No.5,882,680)

By using the above described manufacturing processing method as per US patent No.5,882,680 for multiparticulate seamless minicapsules, nifidipine multiparticulate seamless minicapsules were produced. The completed seamless minicapsules preferably have an average diameter of 0.50 - 5.00mm,more especially in the range 1.50-1.80mm.

According to one embodiment a portion or all of the sustained release particles further comprise an immediate release coating applied onto the rate-controlling polymer coat, which immediate release coating comprises solubilised Methylxanthine or Corticosteroid in a liquid phase.

In an alternative embodiment, the formulation can contain a portion of immediate release minicapsules each comprising a core of a Methylxanthine or a Corticosteroid solubilised in a liquid phase.

The formulation according to the invention may also comprise at least two populations of sustained release seamless minicapsules having two different in vitro dissolution profiles.

Also preferably, the formulation according to the invention provides a dissolution profile in a pre-selected media such that about 0-25% of the combined product is released after 1 hour; about >25% after 3 hours; about >50% after 6 hours; >80 after 12 hours.

In an alternative embodiment the formulation provides a dissolution profile in a pre-determined media such that about <15% of the combined product is released after 1 hour; about >15% is released after 3 hours;> 35% is release after 9 hours; about >45% is released after 12 hours and at least 80% is released after 24 hours.

In a preferred embodiment greater than 80% of the formulation is comprised of sustained release seamless minicapsules.

In a preferred embodiment 50% of the formulation is comprised of a population of sustained release seamless minicapsules of a Methylxanthine, in this case Theophylline, pentoxifylline or A802715 and a population of 50% of a sustained release seamless minicapsule of a Corticosteroid, in this case either Dexamethasone, Prednisolone, Prednisone or Budesonide.

In a preferred embodiment the ratio of seamless minicapsules of a Methylxanthine: Corticosteriod can be formulated in the following ratio's:- 95:5; 90:10; 80:20;70:30; 60:40;50:50 and so on.

In a preferred embodiment the rate-controlling polymer coat contains Ammonia Methacrylate Copolymer Type A and Ammonia Methacrylate Copolymer Type B as described in USP/NF.

Such copolymers are manufactured and marketed by Rohm, GmbH, Darmstadt, Germany.

Most preferably the rate-controlling polymer coat contains a 5:95 or 10:90 or 15:85 mixture of Eudragit RL:Eudragit RS most especially Eudragit RL 12.5:Eudragit RS 12.5 or Eudragit RL30D:Eudragit RS30D or Eudragit E100 or Eudragit E PO or a combination thereof.

Preferably the sustained release seamless minicapsules following application of the rate-controlling polymer coat are dried at a temperature of about 35-55 deg C for between 12-24 hours

In a preferred embodiment the formulation is encapsulated, for example in a hard gelatin capsule.

The sustained release seamless minicapsules are formed by coating the active seamless minicapsule with the rate-controlling polymer coat comprised of ammonio methacrylate copolymers such as those sold under the Trade Mark EUDRAGIT.

EUDRAGIT polymers are polymeric lacquer substances based on acrylates and/or methacrylates. The polymeric materials sold under the Trade Mark EUDRAGIT RL and EUDRAGIT RS are acrylic resins comprising copolymers of acrylic and methacrylic acid esthers with a low content of quaternary ammonium groups and are described in the "EUDRAGIT" brochure of Degussa GmbH wherein detailed physical-chemical data of these products are given. The ammonium groups are present as salts and give rise to the permeability of the lacquer films. EUDRAGIT RL is freely permeable or RS slightly permeable, independent of pH.

Minicapsules with both pH- and time-controlled release (double coating with EUDRADIT® RL/RS and EUDRAGIT® FS30D) may be prepared according to Degussa (In-vivo evaluation of EUDRAGIT™ - A novel pH- and time-controlled multiple unit colonic drug delivery system, Skalsky B., et al, Controlled Release Society 31st Annual Meeting TRANSACTIONS).

The mucoadhesive controlled GIT transit minicapsules are formed by coating the active seamless minicapsule with the transit-controlling polymer coat comprised of, for example various cellulose or cellulose derivatives such as chitosan or those sold under the brand name Carbapol®.

The rate-controlling polymer coat maybe built up by applying a plurality of coats of polymer solution or suspension to the minicapsule as hereafter described. The polymer solution or suspension contains the polymer(s) dissolved or suspended, respectively in a suitable aqueous or organic solvent or mixture of solvents, optionally in the presence of a lubricant. Suitable lubricants are talc, stearic acid, magnesium stearate and sodium stearate. A particularly preferred lubricant is talc.

The polymer solution or suspension may optionally include a plasticizing agent. Suitable plasticizing agents include polyethylene glycol, propyleneglycol, glycerol, triacetin, dimethyl phthalate, diethyl phthalate, dibutyl phthalate, dibutyl sebacate or varying percentages of acetylated monoglycerides.

Suitable organic solvents include isopropyl alcohol (IPA) or acetone or a mixture.

The polymer solution or suspension maybe applied to the minicapsules preferably using an automated system such as a GLATT fluidised bed processor, Vector Flow Coater System or an Aeromatic or a Vector Granurex Rotor Processor System.

Polymer solution/suspension in the quantity of 5-75 ml per kilogram of minicapsules maybe applied to the minicapsules using one of the listed automated fluidised bed processing systems to given target polymer coating weight.

In accordance with the invention the drug loaded minicapsules are coated with the rate-controlling polymers to achieve a target dissolution rate. The drug released from these minicapsules is diffusion controlled as the polymer swells and becomes permeable, it allows for the controlled release in the GIT. In order to achieve a suitable dissolution profile, the following parameters require consideration, efficient process/conditions, drug solubility/particle size, minicapsule surface area, minicapsule diameter and coating polymer suitability.

### Example 1

| Core Solution | |
|---|---|
| -- Corticosteroid(Dexamethasone, Prednisolone, Prednisone or Budesonide) | |
| | 50-200 grams |
| --PEG 400 | 50-500 grams |
| -- Ethanol | 0-500 grams |
| --Vegetable or Mineral Oil | 1000 grams |

| Film Solution | |
|---|---|
| -- Methylxanthine (e.g. Theophylline) | 30-50 %/wt |
| -- Gelatin | 18 %/wt |
| -- Sorbitol | 2 %/wt |
| -- Purified Water | as required |

| Polymer Coating Solution | |
|---|---|
| -- Eudragit RL | 5% w/w |
| -- Eudragit RS | 95% w/w |
| -- Talc | as required |
| -- Minicapsule diameter | 1.50 - 2.00mm |

The Methylxanthine and the Corticosteroid Combination Multiparticulate Seamless Minicapsules were manufactured according to Freund Industrial Co. Ltd. US Patent No. 5,882,680 (Seamless Capsule and Method of Manufacturing Same) and as described in the Summary of the Invention Section.

In order to coat the core seamless minicapsules, a coating solution of 6.25% Eudragit RL (5% w/w) and Eudragit RS (95% w/w) dissolved in isopropyl alcohol/acetone mixture was sprayed onto the minicapsules using an automated fluidised bed processor. Talc was added simultaneously to avoid agglomeration.

The coated minicapsules were dried in an environmentally controlled drier at 35-55 deg C for between 12-24 hours to remove any residual solvents and enhance the functionality of the Eudragit coating.

Encapsulation 10-90% immediate release: 10-90% sustained release, most preferably a 50:50 ratio.

Methylxanthine/Corticosteroid seamless minicapsules uncoated (50% w/w by potency) and the polymer coated minicapsules (50% w/w by potency) from the above were blended using a suitable mechanical blender.

The resultant blend was filled into suitable gelatin capsules to the required target strength.

### Example 2

| Core Solution | |
|---|---|
| -- Methlyxanthine (Theophylline, pentoxifylline or A802715 USP/EP) | 800 grams |
| -- Gelatin | 1100 grams |
| -- Sorbitol | 100 grams |
| -- Purified Water | 4200 grams |

| Polymer Coating solution | |
|---|---|
| -- Eudragit RS | 95% w/w |
| -- Eudragit RL | 5% w/w |
| -- Diethylphthalate | 5-10% w/w |
| -- Talc | as required |
| -- Minicapsule Diameter | 1.50 - 2.00mm |

The above seamless minicapsules were manufactured in the same way as Example 1 with the following exceptions:-
1. The Methylxanthine was added into the core solution and was treated with a High Pressure Homogeniser.
   The median and film solutions were excluded from this example.
2. The polymer solution included a 5-10% plasticiser.

### Example 3

| Core Solution | |
|---|---|
| -- Corticosteroid (Dexamethasone, Prednisolone, Prednisone or Budesonide) | |
| | 5-50 %/wt |
| -- PEG (200;300;400;600) | 50-95 %/wt |
| -- MCT (Medium Chain Fatty Acid Trigliceride) | 100 %/wt |

| Film Solution | |
|---|---|
| -- Gelatin | 10 - 25 %/wt |
| -- Sorbitol | 1 -5 %/wt |
| -- Purified Water | 50 -100 %/wt |

| Polymer Coating Solution | |
|---|---|
| -- Eudragit S | 100 %/wt |
| -- Isopropyl Alcohol/acetone | as required |
| -- Talc | as required |
| -- Minicapsule Diameter | 1.50 - 2.00mm |

The above seamless minicapsules were manufactured in the same way as Example 1 with the following exceptions:-
1. The core solution was pre-treated with an Ultra Centrifugal Mill.
2. Eudragit S was used as the polymer coat to provide an enteric coat with 0 drug release of up to 2-6 hours to the minicapsules, to target the drug release to the GIT and providing a pulsed release profile.

A percentage of the Enteric Coated Corticosteroid (Example 3) seamless minicapsules and a percentage of the coated Methylxanthine seamless minicapsules from Example 2 were blended as per in Example 1 and filled into suitable gelatin capsules to the target strength.

### Example 3a

Core and film is as per Example 3.

| Mucoadhesive Coating Solution | |
|---|---|
| -- Ethylcellulose | 5-20g |
| -- PVP | 0.5-5g |
| -- Castor Oil | 0-5g |
| -- Magnesium Stearate | 0.5-3g |
| -- Aceton | 50-300g |
| -- Isopropanol | 5-50g |

| Polymer Coating Solution | |
|---|---|
| -- Eudragit RL | 5% w/w |
| -- Eudragit RS | 95% w/w |
| -- Minicapsule diameter | 1.50mm |

The Corticosteroid Multiparticulate Seamless Minicapsules were manufactured according to Freund Industrial Co. Ltd US Patent No. 5,882,680 (Seamless Capsule and Method of Manufacturing Same) and as described in the Summary of the Invention Section.

To apply a mucoadhesive coating, a coating solution of 7.0% ethylcellulose, 0.85% PVP and 1.0% Magnesium Stearate was dissolved in an isopropanil/acetone mixture was sprayed onto the minicapsules using an automated fluidised bed processor. Anti-agglomeration powder was applied to prevent agglomeration of the minicapsules. The coated minicapsules were dried in an environmentally controlled drier for between 12 to 24 hours to remove any residual solvents

In order to further coat the mucoadhesive coated seamless minicapsules, a coating solution of 6.25% Eudragit RL (5% w/w) and Eudragit RS (95% w/w) dissolved in isopropyl alcohol/acetone mixture was sprayed onto the minicapsules using an automated fluidised bed processor. Talc was added simultaneously to avoid agglomeration.

The coated minicapsules were dried in an environmentally controlled drier for between 12 to 24 hours to remove any residual solvents

Encapsulation 0-100% immediate release/100-0% sustained release.

Corticosteroid seamless minicapsules uncoated (10% w/w by potency) and the polymer coated minicapsules (90% w/w by potency) from the above were blended using a suitable mechanical blender as per Example 3. The resultant blend was filled into suitable gelatin capsules or sprinkle format to the required target strength.

### Example 4

| Core Solution | |
|---|---|
| -- Corticosteroid (Dexamethasone, Prednisolone or Prednisone) | 50-200 grams |
| -- PEG 400 | 50-500 grams |
| -- Ethanol | 0-500 grams |
| --Vegetable or Mineral Oil | 1000 grams |

| Film Solution | |
|---|---|
| -- Methylxanthine (e.g. Theophylline) | 30-50 %/wt |
| -- Gelatin | 18 %/wt |
| -- Sorbitol | 2 %/wt |
| -- Purified Water | as required |

| Median Solution | |
|---|---|
| --Vegetable or Mineral Oil | 1000 grams |

| Film Solution | |
|---|---|
| -- Gelatin | 225 grams |
| -- Sorbitol | 25 grams |
| -- Purified Water | 750 grams |

| Mucoadhesive Coating Solution | |
|---|---|
| -- Ethylcellulose | 5-20g |
| -- PVP | 0.5-5g |
| -- Castor Oil | 0-5g |
| -- Magnesium Stearate | 0.5-3g |
| -- Aceton | 50-300g |
| -- Isopropanol | 5-50g |

| Rate-Release Polymer Coating Solution | |
|---|---|
| -- Eudragit RL | 5% w/w |
| -- Eudragit RS | 95% w/w |
| -- Minicapsule diameter | 1.50mm |

The Methylxantine and the Corticosteroid Combination Multiparticulate Seamless Minicapsules were manufactured according to Freund Industrial Co. Ltd. US Patent No. 5,882,680 (Seamless Capsule and Method of Manufacturing Same) and as described in the Summary of the Invention Section.

In order to coat the core seamless minicapsules, a coating solution of 6.25% Eudragit RL (5% w/w) and Eudragit RS (95% w/w) dissolved in isopropyl alcohol/acetone mixture was sprayed onto the minicapsules using an automated fluidised bed processor. Talc was added simultaneously to avoid agglomeration.

To apply a mucoadhesive coating, a coating solution of 7.0% ethylcellulose, 0.85% PVP and 1.0% Magnesium Stearate was dissolved in an isopropanil/acetone mixture was sprayed onto the minicapsules using an automated fluidised bed processor. Anti-agglomeration powder was applied to prevent agglomeration of the minicapsules. The coated minicapsules were dried in an environmentally controlled drier for between 12 to 24 hours to remove any residual solvents

In order to further coat the mucoadhesive coated seamless minicapsules, a coating solution of 6.25% Eudragit RL (5% w/w) and Eudragit RS (95% w/w) dissolved in isopropyl alcohol/acetone mixture was sprayed onto the minicapsules using an automated fluidised bed processor. Talc was added simultaneously to avoid agglomeration.

The coated minicapsules were dried in an environmentally controlled drier at 35-55 deg C for between 12-24 hours to remove any residual solvents and enhance the functionality of the Eudragit coating.

Encapsulation 10-90% immediate release: 10-90% sustained release, most preferably a 50:50 ratio.

Methylxanthine/Corticosteroid seamless minicapsules uncoated (50% w/w by potency) and the polymer coated minicapsules (50% w/w by potency) from the above were blended using a suitable mechanical blender.

The resultant blend was filled into suitable gelatin capsules to the required target strength.

The invention is not limited to the embodiments hereinbefore described which may be varied in detail.

### Anti cancer agents in combination with P-gp/P450 inhibitors

The development of multidrug resistance remains one of the most serious impediments to effective, curative chemotherapy in cancer patients. Resistance develops from a cancer cell's natural response to anticancer drugs. It is believed that by understanding these cellular responses and the mechanisms of action of specific drugs that more effective therapeutics and/or treatment protocols can be developed. Drug resistance to cancer agents such as vinblastine, docetaxel and palitaxel is often as a result of the efflux action of the plasma membrane protein drug pump, P-gp or due to the oxidative metabolism of the anti-cancer agent by cytochrome P450 in the gut wall.

Although it is possible to employ some commonly used excipients, including surfactants and oils to inhibit these efflux and degradative pathways, it is often more effective to administer a combination of the anti-cancer agent with another drug which acts as a P-gp/P450 inhibitor. Among the inhibitors commonly used are cyclosporine A, omerprazole, verapamil and its non-toxic enantiomers.

### Example 5

Tamoxifen, an anti-estrogen is used in the treatment of breast cancer. Drug resistance eventually limits the effectiveness of antiestrogens in breast cancer treatment. Pharmacological reversal of this refractoriness has been attempted with R-verapamil, a well tolerated calcium channel blocker. It has been shown that the simultaneous administration of antiestrogens (tamoxifen) with a non-toxic enantiomer of verapamil (R- verapamil) significantly decreases tumour growth, a fact that has been correlated with reduction in the expression of P-glycoprotein (due to the inhibitory action of the R-verapamil), (Anticancer Res., 1991, 11, 809-1.

A tamoxifen SEDDS (Self Emulsifying Drug Delivery System) formulation is prepared with polyoxyl hydrogenated castor oil. A formulation consisting of a modified vegetable oil (e.g., polyoxyl hydrogenated castor oil), a surfactant (e.g., TPGS), a co-solvent (e.g., propylene glycol) and a bile salt (e.g., sodium deoxycholate) is prepared by successive addition and mixing of each component. The tamoxifen is then added to the formulation, which is thoroughly mixed to form a clear homogenous mixture. The R-verapamil is finally added and dissolved quickly under mild agitation. The tamoxifen/R-verapamil pre-microemulsion concentrate is then formed into seamless microcapsules according to the methods described in US Pat. Nos. 5, 478,508 and 5,882,680 with an intermediate oil layer and an outer gelatin shell.

### Core Formulation

| Ingredients | % w/w |
|---|---|
| Tamoxifen | 2.5 |
| R-verapamil | 2.5 |
| Unconjugated deoxycholic acid | 5 |
| Fractionated oat oil | 30 |
| Cremophor EL or TPGS | 30 |
| PEG 400 | 30 |

### Intermediate Solution

| | |
|---|---|
| Vegetable oil | 100 |

### Shell Solution

| Ingredients | % w/w |
|---|---|
| Gelatin | 15-20 |
| Sorbitol/Glycerin | 1-5 |
| Purified Water | 70-80 |

### Example 5a

Sustained release tamoxifen/R-verapamil minicapsules may also be formulated by coating the seamless minicapsules (described in Example 5), with the rate-controlling polymer coat comprised Eudragit RS and Eudragit RL. The formulation for the Eudragit RL (5% w/w) and Eudragit RS (95% w/w) coating solution is outlined below.

### Sustained Release Polymer Coating Solution

| Ingredients | % w/w |
|---|---|
| Eudragit RL 100 (5%) /Eudragit RS 100 (95%) | 10-15 |
| Acetone | 30-35 |
| Isopropyl alcohol | 50-60 |
| Water | 2.5-5.0 |

### Example 5b

Sustained release antiestogen analogues (including tamoxifen, 4-hydroxy-tamoxifen (4OHT), idoxifene, raloxifene, GW7604, and ICI 182,780) / R-verapamil metalbolites and enantiomers (including verapamil, (R)-norverapamil, (S)-norverapamil, racemic norverapamil, PR-22, or D-620) minicapsules may also be formulated as described in Example 5 and furhter by coating the seamless minicapsules (described in Example 5), with the rate-controlling for polymer coat comprised Eudragit RS and Eudragit RL. The formulation and process for the Eudragit RL (5% w/w) and Eudragit RS (95% w/w) coating solution is described in Example 5a).

### Example 6

As illustrated above, it is also possible to incorporate drug combinations in SEDDS/SMEDDS/SMEOFS formulations. Although many oral formulations of the invention will provide therapeutic blood levels of the active ingredient when administered alone, it is sometimes beneficial to co-formulate a second active into the formulation to improve efficacy. This method is of particular use when the primary active is a substrate for P-glycoprotein. The taxanes (e.g., Paclitaxel and Docetaxel) are an example of drugs which are inhibited by P-gp and therefore will benefit from the co formulation of a bioenhancing agent for example cyclosporine (Malingré et al., (2001). Coadministration of Cyclosporine Strongly Enhances the Oral Bioavailability of Docetaxel. Journal of Clinical Oncology, 19, 1160-1166.)

The procedure is the same as that followed in Example 5 with paclitaxel/docetaxel being added instead of the tamoxifen and the cyclosporine A being added instead of the R-verapamil. The paclitaxel (docetaxel)/cyclosporine pre-microemulsion concentrate is then formed into seamless microcapsules according to the methods described in US Pat. Nos. 5, 478,508 and 5,882,680 with an intermediate oil layer and an outer gelatin shell. The intermediate layer and shell formulation are as Example 5.

### Core Formulation

| Ingredients | % w/w |
|---|---|
| Paclitaxel/Docetaxel | 5 |
| Unconjugated deoxycholic acid | 5 |
| Fractionated oat oil | 30 |
| Cremophor EL or TPGS | 30 |
| PEG 400 | 30 |

### Example 6a

Sustained release paclitaxel (docetaxel)/cyclosporine A minicapsules may also be formulated by coating the seamless minicapsules (described in Example 2), with the rate-controlling polymer coat comprised Eudragit RS and Eudragit RL. The formulation for the Eudragit RL (5% w/w) and Eudragit RS (95% w/w) coating solution is the same as that outlined in Example 5a.

### Anti-HIV/AIDS agents in combination with P-gp/P450 inhibitors

P-glycoprotein functions as a membrane-localized drug transport mechanism that has the ability to actively pump out all currently prescribed HIV-protease inhibitors (PIs) from the intracellular cytoplasm. This effect may result in limited oral bioavailability of PIs and limited accumulation in P-gp expressing cells as well as a decreased ability to cross blood-tissue barriers such as the blood-brain barrier, the blood-testis barrier and the materno-fetal barrier. MDR1 encoded P-glycoprotein has also been implicated in the cytotoxicity process and in the induction of immune responses during HIV infection. HIV PIs, which are substrates for P-gp include; amprenavir (APV), indinavir (IDV), nelfinavir (NFV), ritonavir (RTV) and saquinavir (SQV). Similarly to the immunosuppressant cyclosporine A, many of these HIV PIs (with the possible exception of IDV) also act as P-gp inhibitors. Many of the HIV PIs that are transported by P-gp are metabolized by some of the cytochromes (CYPs), especially CYP450 3A which constitutes 30% and 70% of the total CYP450s in most human livers and intestines, respectively. The effect of P-gp and cytochrome P450 on limiting oral bioavailability and tissue distribution of PIs has obvious implications for the effectiveness of PI-containing regimens. It is necessary to formulate a combination LEDDS to include a P-gp/P450 inhibitor in the formulation. P-gp transport of HIV PIs can be inhibited by P-gp inhibitors like cyclosporine A, quinidine, verapamil, and PSC833 (AIDS, 2000, 14, 235-6).

### Example 7

Saquinavir and R-verapramil are solubilised/suspended in a suitable medium chain triglyceride (MCT) and formed into seamless microcapsules with an outer gelatin coating according to the methods described in US Pat. Nos. 5, 478,508 and 5,882,680. The core formulation is outlined below. The shell formulation is the same as that outlined in Example 5.

### Core Formulation

| Ingredients | % w/w |
|---|---|
| Saquinavir | 5-10 |
| Cyclosporine A | 5-10 |
| MCT | 80-90 |

Alternatively the saquinavir and the cyclosporine A may be formulated as separate minicapusles by again solubilising/suspending in a suitable medium chain triglyceride (MCT) and forming into seamless microcapsules with an outer gelatin coating according to the methods described in US Pat. Nos. 5, 478,508 and 5,882,680. The two populations of saquinavir and cyclosporine A minicapsules, are then blended together and filled into a hard gelatin capsule or sachet for administration.

### Example 7a

The saquinavir/cyclosporine minicapsule combination may be gastro-protected by coating the seamless minicapsules (described in Example 7), with the enteric polymer, Eudragit S 12.5, providing zero drug release in the stomach up to 4 hours. The formulation for the Eudragit S 12.5 coating solution is outlined below.

### Enteric Polymer Coating Solution

| Ingredients | % w/w |
|---|---|
| Eudragit S 12.5 | 50 |
| Triethyl citrate | 1-5 |
| Talc | 10-15 |
| Isopropyl alcohol | 40-45 |

### Example 8

It is also possible to formulate a combination of two HIV PIs, for example, a ritonavir/ lopinavir combination. In this formulation, the ritonavir acts as a potent inhibitor of the hepatic cytochrome P450 isoenzyme CYP3A, thereby decreasing the metabolism of and increasing the plasma concentrations of lopinavir. Ritonavir and lopinavir are solubilised/suspended in a suitable medium chain triglyceride (MCT) and formed into seamless microcapsules with an outer gelatin coating according to the methods described in US Pat. Nos. 5, 478,508 and 5,882,680. The core formulation is outlined below. The shell formulation is the same as that outlined in Example 5.

### Core Formulation

| Ingredients | % w/w |
|---|---|
| Ritonavir | 5-10 |
| Lopinavir | 5-10 |
| MCT | 80-90 |

### Example 8a

The ritonavir/lopinavir minicapsule combination may be gastro-protected by coating the seamless minicapsules (described in Example 8), with the enteric polymer, Eudragit S 12.5, providing zero drug release in the stomach up to 4 hours. The formulation for the Eudragit S 12.5 coating solution is the same as that described in Example 7a.

### Anti-Malarial combinations

Early and effective chemotherapy for malaria has a pivotal role in reducing morbidity and mortality especially since a vaccine is unlikely to emerge within the next decade. Multidrug resistance has been reported from most parts of the world and as a result, monotherapy or some of the available combination chemotherapies for malaria are either ineffective or less effective. New antimalarial regimens are, therefore, urgently needed and antimalarial combination chemotherapy is widely advocated. Antimalarial combinations can increase efficacy, shorten duration of treatment (and hence increase compliance), and decrease the risk of resistant parasites arising through mutation during therapy. Combination therapy with antimalarial drugs is the simultaneous use of two or more blood schizontocidal drugs with independent modes of action and different biochemical targets in the parasite. The concept of combination therapy is based on the synergistic or additive potential of two or more drugs, to improve therapeutic efficacy and also delay the development of resistance to the individual components of the combination. Artemisinin based combinations (e.g. Artesunate) are known to improve cure rates, reduce the development of resistance and they might decrease transmission of drug-resistant parasites. The total effect of artemisinin combinations (which can be simultaneous or sequential) is to reduce the chance of parasite recrudescence, reduce the within-patient selection pressure, and prevent transmission.

### Example 9

Polyethylene glycol is added to purified water (50:50) in a suitable container and mixed using a mechanical mixer. Artesunate is then added to the solution. In a separate container, sulfadoxine/primethamine (SP) is added to vegetable oil. The polyethylene glycol/artesunate solution and the vegetable oil/SP solution are then formed into seamless microcapsules according to the methods described in US Pat. Nos. 5, 478,508 and 5,882,680. The core and intermediate formulations are outlined below. The shell formulation is the same as that outlined in Example 5.

### Core Formulation

| Ingredients | % w/w |
|---|---|
| Artesunate | 10-20 |
| PEG 400 | 80-90 |

### Intermediate Solution

| | |
|---|---|
| Sulfadoxine/primethamine | 10-20 |
| Vegetable oil | 80-90 |

### Anti-TB combinations

### Example 10

Tuberculosis (TB) is treated using a combination of drugs (antibiotics). As with HIV, in which a combination of antiretroviral drugs is often used to help prevent resistance and keep viral load undetectable, tuberculosis is usually treated with a combination of drugs to maintain control over the infection. Some people are infected with strains of *Mycobacterium tuberculosis* that are resistant to one or more of the drugs commonly used to treat tuberculosis, therefore combination therapy is essential for TB treatment. Drugs used in the treatment of tuberculosis include; isoniazid, rifampin and pyrazinamide. It is possible to administer these drugs as a combination of single dosage forms (i.e. three separate tablets etc.), however it is preferable to combine the drugs into one dosage form. The multiparticulate technology (LEDDS) of the invention allows for such a dosage regime. It is possible to formulate the three drugs outlined above (isoniazid, rifampin and pyrazinamide) as individual minicapsules which are later blended and combined into a hard gelatin capsule for oral administration. As there are three actives ingredients included in a single dosage form for oral administration, it is necessary to maximize the loading of the three drugs in their respective minicapsules. This maximum loading is facilitated by incorporating the respective drugs into both the core formulation and the gelatin shell of the minicapsules. The formulations for the three anti-TB drugs are outlined below. The core formulation solutions and the gelatin/drug solutions are then formed into seamless microcapsules according to the methods described in US Pat. Nos.5, 478,508 and 5,882,680.

### Core Solution 1

| Ingredients | % w/w |
|---|---|
| Isoniazid | 10-20 |
| MCT | 80-90 |

### Shell Solution 1

| Ingredients | % w/w |
|---|---|
| Gelatin | 15-20 |
| Sorbitol/Glycerin | 1-5 |
| Isoniazid | 0-10 |
| Purified Water | 70-80 |

### Core Solution 2

| Ingredients | % w/w |
|---|---|
| Rifampin | 10-20 |
| MCT | 80-90 |

### Shell Solution 2

| Ingredients | % w/w |
|---|---|
| Gelatin | 15-20 |
| Sorbitol/Glycerin | 1-5 |
| Rifampin | 0-10 |
| Purified Water | 70-80 |

### Core Solution 3

| Ingredients | % w/w |
|---|---|
| Pyrazinamide | 10-20 |
| MCT | 80-90 |

### Shell Solution 3

| Ingredients | % w/w |
|---|---|
| Gelatin | 15-20 |
| Sorbitol/Glycerin | 1-5 |
| Pyrazinamide | 0-10 |
| Purified Water | 70-80 |

### Example 10a

The isoniazid, rifampin and pyrazinamide minicapsules may be gastro-protected by coating the seamless minicapsules (described in Example 10), with the enteric polymer, Eudragit S 12.5, providing zero drug release in the stomach up to 4 hours. The formulation for the Eudragit S 12.5 coating solution is outlined below. It is also possible to incorporate the three drugs into the polymer coat to further increase the drug loading.

### Enteric Polymer Coating Solution

| Ingredients | % w/w |
|---|---|
| Isoniazid, rifampin and pyrazinamide | 0-10 |
| Eudragit S 12.5 | 50 |
| Triethyl citrate | 1-5 |
| Talc | 10-15 |
| Isopropyl alcohol | 40-45 |

### HIV/Malaria/TB combination

### Example 11

The multiparticulate nature of the LEDDS technology enables the combination of anti-HIV/Malaria/TB drugs in one formulation by formulating the drugs individually in seamless microcapsules as described in Examples 7-10 and then blending, combining and filling the three populations of minicapsule (HIV, Malaria and TB) into one or two hard gelatin capsules depending on the dose required.

### Anti-HIV/AIDS agents in combination with immunostimulators

### Example 12

Immunostimulants represent an emerging class of drugs for the treatment of infectious disorders and cancer. The acyclic nucleoside phosphonates (ANPs) are soluble immune factors that play critical roles in defense mechanisms against infections. Some of these agents are used in clinical practice against various infections, including hepatitis B (adefovir), HIV (tenofovir) and HIV-associated (cidefovir) infections. ANPs have been shown to be potent immunostimulators. The major mechanism of antiviral action of ANPs is the inhibition of virus-induced DNA polymerases and/or reverse transcriptases. They suppress replication of both DNA-viruses and retroviruses. By combining an immunostimulant such as tenofovir with a HIV PI such as amprenavir, a more effective treatment can be obtained.

An oil-in-water emulsion is formed where the poorly soluble amprenavir (10-20% w/w) is solubilised/suspended in the oil phase (mineral oil) and the freely soluble tenofovir (10-20% w/w) is solubilised in the water phase. Both emulsion components are then mixed with the required amount of surfactant (e.g., TPGS). The amprenavir/tenofovir emulsion is then thoroughly mixed to form a clear homogenous mixture. The amprenavir/tenofovir emulsion is then formed into seamless microcapsules according to the methods described in US Pat. Nos. 5, 478,508 and 5,882,680 with an intermediate oil layer and an outer gelatin shell. The formulation for the core emulsion is outlined below. The intermediate and shell layer formulations are the same as that outlined in Example 5.

### Core Formulation

| Ingredients | % w/w |
|---|---|
| Amprenavir/tenofovir | 10-20 |
| Mineral oil | 10-20 |
| Water | 70-80 |
| TPGS | 1-5 |

### Example 12a

Sustained release amprenavir/tenofovir minicapsules may also be formulated by coating the seamless minicapsules (described in Example 12), with the rate-controlling polymer coat comprised Eudragit RS and Eudragit RL. The formulation for the Eudragit RL (5% w/w) and Eudragit RS (95% w/w) coating solution is the same as that outlined in Example 5a.

### Anti-cancer agents in combination with immunostimulators

### Example 13

Isoprinosine, also known as Imunovir is known as an adjuvant treatment for cancer. Patients on chemotherapy are particularly susceptible to different viral infections as a result of chemotherapy-induced immunodepression. Adjuvant therapy with Isoprinosine can restore the cell-mediated immune response to the individual's baseline levels. Isoprinosine acts on the immune system to restore impaired cell-mediated response to baseline in addition to enhancing humoral response. It has also a direct antiviral activity. Isoprinosine can reduce the intensity of symptoms and shorten the duration of the viral infection. In addition, the occurrence of complications is reduced and the frequency and severity of recurrences is minimised. By combining the immunostimulatory effects of the imunovir and an anti- cancer agent such vinblastine, a superior anti-cancer therapy can be formulated. It is also necessary to include a P-gp blocker such as R -verapramil or verapamil metabolite in the formulation. The vinblastine and R- verapramil or verapamil metabolite is formulated as a microemulsion. The method is the same as that described in Example 5. The Isoprinosine is formulated in separate minicapsules, with the active solubilised/suspended in a medium chain triglyceride in the minicapsule core. The gelatin shell formulation for both types of minicapsule is the same as that outlined in Example 5 (the vinblastine/R-verapramil microemulsion is also encapsulated by an intermediate vegetable oil layer).

### Minicapsule Core Formulation 1

| Ingredients | % w/w |
|---|---|
| Vinblastine | 2.5 |
| R-verapamil (enantiomer or metabolite) | 2.5 |
| Unconjugated deoxycholic acid | 5 |
| Fractionated oat oil | 30 |
| Cremophor EL or TPGS | 30 |
| PEG 400 | 30 |

### Minicapsule Core Formulation 2

| Ingredients | % w/w |
|---|---|
| Isoprinosine | 10-20 |
| MCT | 80-90 |

The two populations of minicapsules (1 & 2) are then blended and filled into hard gelatin capsules depending on the dose required.

### Example 13a

Sustained release Isoprinosine and Vinblastine/R-verapramil minicapsules may also be formulated by coating the seamless minicapsules (described in Example 13), with the rate-controlling polymer coat comprised Eudragit RS and Eudragit RL. The formulation for the Eudragit RL (5% w/w) and Eudragit RS (95% w/w) coating solution is the same as that outlined in Example 5a.

### Anti-cancer agents in combination with chemosensitizers

### Example 14

Isoflavones or isoflavanoids, including phenoxodiol have been shown to induce cell death in taxane or platinum-resistant cancer cells. (Phenoxodiol - an isoflavone analogue - induces apoptosis in chemoresistant ovarian cancer cells; Kamsteag et al., Oncogene (2003), 22, 2611-2620). A major issue with cancer chemotherapy is that patients develop a resistance to the chemotherapeutic agent, thus requiring larger doses, leading to immunosuppression and other toxic side effects, including nephrotoxicity. Adjuvant co-therapy with phenoxodiol can restore chemosensitivity to chemoresistant tumour cells. By combining the chemosensitizer effects of the phenoxodiol or similar with an anti- cancer agent such as a taxane (paclitaxel, taxotere or similar), a superior anti-cancer therapy can be formulated. It is also necessary to include a P-gp blocker such as R -verapramil or verapamil metabolite in the formulation. The vinblastine and R- verapramil or verapamil metabolite is formulated as a microemulsion. The method is the same as that described in Example 5. The isoflavone, phenoxodiol, is formulated in separate minicapsules, with the active solubilised/suspended in a medium chain triglyceride in the minicapsule core. The gelatin shell formulation for both types of minicapsule is the same as that outlined in Example 5 (the vinblastine/R-verapramil microemulsion is also encapsulated by an intermediate vegetable oil layer).

### Minicapsule Core Formulation 1

| Ingredients | % w/w |
|---|---|
| Paclitaxel | 10 |
| Phenoxodiol | 10 |
| R-verapamil (enantiomer or metabolite) | 2.5 |
| Unconjugated deoxycholic acid | 5 |
| Fractionated oat oil | 25 |
| Cremophor EL or TPGS | 25 |
| PEG 400 | 25 |

### Minicapsule Core Formulation 2

| Ingredients | % w/w |
|---|---|
| Phenoxodiol | 10-20 |
| MCT | 80-90 |

The two populations of minicapsules (1 & 2) are then blended and filled into hard gelatin capsules depending on the dose required.

### Tamper-proofing combinations

### Example 14a

Oxycodone is used to relieve moderate to moderate-to-severe pain. Oxycodone abuse has been a continuing problem, similar to other opioids, is abused for its euphoric effects. It is equipotent to morphine in relieving abstinence symptoms from chronic opiate (heroin, morphine) administration. As a result of the problems associated with oxycodone abuse, it has become necessary to develop a combination formulation, in which the pain killer is made unsuitable for drug abuse. One such approach is the co-administration of the pain killer with an irritant, with the irritant being released only when the drug is abused. The LEDDS technology allows for a combination of oxycodone hydrochloride minicapsules with capsaicin (irritant) minicapsules. The capsaicin minicapsules are coated appropriately so that the minicapsules will pass through the intestine and be excreted intact. Should the oxycodone/capsaicin formulation be ruptured for the purposes of drug abuse (oxycodone/capsaicin injected into bloodstream), then the capsaicin will cause discomfort to the abuser.

Oxycodone hydrochloride is dissolved in a low viscosity MCT and formed into seamless microcapsules according to the methods described in US Pat. Nos. 5, 478,508 and 5,882,680. The shell formulation is the same as that outlined in Example 5. A sustained release coating is applied to the oxycodone hydrochloride minicapsules using the same formulation and procedure as described in Example 5a.

### Core Formulation

| Ingredients | % w/w |
|---|---|
| Oxycodone Hydrochloride | 10-20 |
| Low Viscosity MCT | 80-90 |

Capsaicin is dissolved in a low viscosity MCT and formed into seamless microcapsules according to the methods described in US Pat. Nos. 5, 478,508 and 5,882,680. The shell formulation is the same as that outlined in Example 1. Two layers of enteric coating are applied to the capsaicin minicapsules. Eudragit E PO (soluble at acidic pH) is initially applied to the minicapsules. Once the initial coat is dry, a second polymer coat of Eudragit S 12.5 (dissolves at pH 7) is applied. The formulation of the coating solution for the Eudragit E PO is outlined below. The formulation for the Eudragit S 12.5 coating solution is the same as that outlined in Example 3a. The procedures for applying the polymer coats are the same as that given in Example 1a. As the capsaicin minicapsules progress down the GIT to the colon, the outer enteric coating will be dissolved, revealing the inner coat, which requires an acidic pH to be dissolved. Therefore the irritant can pass freely through the GIT before being excreted intact.

### Core Formulation

| Ingredients | % w/w |
|---|---|
| Capsaicin | 10-20 |
| Low Viscosity MCT | 80-90 |

### Eudragit E PO Polymer coat

| | |
|---|---|
| Eudragit E PO | 10-15 |
| Sodium lauryl sulphate | 0-1 |
| Stearic acid | 0-2.5 |
| Magnesium state | 2-5 |
| Demineralised water | 80-90 |

### Combination of nimodipine and P-gp/P450 inhibitors

### Example 15

Nimodipine is metabolized through the cytochrome P450 system. By combining nimodipine with the cytochrome P450 inhibitor, carbamazepine (anticonvulsant), the clinical effectiveness of nimodipine may be increased (Clin Psychopharmacol., 1998, 18, 404-13). A nimodipine SEDDS (Self Emulsifying Drug Delivery System) formulation is prepared with polyoxyl hydrogenated castor oil. A formulation consisting of a modified vegetable oil (e.g., polyoxyl hydrogenated castor oil), a surfactant (e.g., TPGS), a co-solvent (e.g., propylene glycol) and a bile salt (e.g., sodium deoxycholate) is prepared by successive addition and mixing of each component. The nimodipine is then added to the formulation, which is thoroughly mixed to form a clear homogenous mixture. The carbamazepine is finally added and dissolved quickly under mild agitation. The nimodipine/carbamazepine pre-microemulsion concentrate is then formed into seamless microcapsules according to the methods described in US Pat. Nos. 5, 478,508 and 5,882,680 with an intermediate oil layer and an outer gelatin shell. The formulation for the intermediate oil layer and outer gelatin shell are the same as that outlined in Example 5.

### Core Formulation

| Ingredients | % w/w |
|---|---|
| Nimodipine | 2.5-5 |
| Carbamazepine | 2.5 |
| Unconjugated deoxycholic acid | 5 |
| Fractionated oat oil | 30 |
| Cremophor EL or TPGS | 30 |
| PEG 400 | 30 |

### Example 15a

Sustained release nimodipine/carbamazepine minicapsules may also be formulated by coating the seamless minicapsules (described in Example 11), with the rate-controlling polymer coat comprised Eudragit RS and Eudragit RL. The formulation and coating procedure for the Eudragit RL (5% w/w) and Eudragit RS (95% w/w) is the same as that outlined in Example 5a.

### Example 16

Another anticonvulsant, valproic acid, has also been shown to inhibit the presystemic oxidative metabolism of nimodipine, resulting in increased plasma concentrations of nimodipine when the two drugs are administered in combination (Drugs Aging, 1995, 6, 229-42).

A nimodipine/valproic acid SEDDS (Self Emulsifying Drug Delivery System) formulation is prepared with polyoxyl hydrogenated castor oil as described in Example 11 above, with the valproic acid replacing the carbamazepine in the formulation. The nimodipine/valproic minicapsules may be coated with a Eudragit RS and Eudragit RL polymer coat as described in Example 15a.

### Example 17

The antihistamine, cimetidine, has also been shown to produce an approximate doubling of the bioavailability of nimodipine, as a result of the known inhibitory effect of cimetidine on cytochrome P450 (Drugs Aging, 1995, 6, 229-42).

A nimodipine/ cimetidine SEDDS (Self Emulsifying Drug Delivery System) formulation is prepared with polyoxyl hydrogenated castor oil as described in Example 11 above, with the cimetidine replacing the carbamazepine in the formulation. The nimodipine/ cimetidine minicapsules may be coated with a Eudragit RS and Eudragit RL polymer coat as described in Example 11 a.

### Combination of ramipril and rosiglitazone for diabetes therapy

### Example 18

Ramipril belongs in a class of drugs called angiotensin converting enzyme (ACE) inhibitors which are used for treating high blood pressure and heart failure and for preventing kidney failure due to high blood pressure and diabetes. ACE is important because it produces the protein, angiotensin II. Angiotensin II contracts the muscles of most arteries in the body, including the heart, thereby narrowing the arteries and elevating the blood pressure. In the kidney, the narrowing caused by angiotensin II also increases blood pressure and decreases the flow of blood. ACE inhibitors such as ramipril lower blood pressure by reducing the production of angiotensin II, thereby relaxing the arterial muscles and enlarging the arteries. In the kidneys, the enlargement of the arteries also reduces blood pressure and increases blood flow. Ramipril slows the progression of kidney failure in patients with diabetes.

Rosiglitazone is a drug that reduces the amount of glucose (sugar) in the blood. It is in a class of anti-diabetic drugs called "thiazolidinediones" that are used in the treatment of type II diabetes. Since both drugs are effective in the treatment of diabetes, it would be preferable to combine both drugs in a singular dosage form. LEDDS provides the technology to allow for such a combination.

Polyethylene glycol is added to purified water (50:50) in a suitable container and mixed using a mechanical mixer. Since rosiglitazone is soluble in a buffered aqueous (acidic) solution, a suitable quantity of citric acid is added to the PEG/water mix, to bring the solution to a predetermined pH value. Rosiglitazone is so is then added to the solution. In a separate container, ramipril (highly lipophilic) is added to vegetable oil. The rosiglitazone solution and the vegetable oil/ ramipril solution are then formed into seamless microcapsules according to the methods described in US Pat. Nos. 5, 478,508 and 5,882,680. The core and intermediate formulations are outlined below. The shell formulation is the same as that outlined in Example 5.

### Core Formulation

| Ingredients | % w/w |
|---|---|
| Rosiglitazone | 10-20 |
| Anhydrous citric acid (pH Adjuster) | |
| PEG 400/water | 80-90 |

### Intermediate Solution

| | |
|---|---|
| Ramipril | 10-20 |
| Vegetable oil | 80-90 |

### Example 18a

The rosiglitazone/ramipril minicapsule combination may be gastro-protected by coating the seamless minicapsules (described in Example 14), with the enteric polymer, Eudragit L 30 D-55, providing zero drug release in the stomach up to 4 hours. Eudragit L 30 D-55 is insoluble in acid media, but dissolves above pH 5.5 and is used because of the fact that the solubility of rosiglitazone decreases with increasing pH in the physiological range. The formulation for the Eudragit L 30 D-55 coating solution is outlined below. The coating procedure is the same as that outlined in Example 5a.

### Enteric Polymer Coating Formulation

| Ingredients | % w/w |
|---|---|
| Eudragit L 30 D-55 | 50-60 |
| Talc | 5-10 |
| Triethyl citrate | 1-5 |
| Water | 35-45 |

### Combinations of the statins with angiotensin converting enzyme (ACE) inhibitors

### Example 19

Simvastatin is a cholesterol- lowering medicine. It inhibits the production of cholesterol by the liver. It lowers overall blood cholesterol as well as blood LDL cholesterol levels. LDL cholesterol is believed to be the "bad" cholesterol that is primarily responsible for the development of coronary artery disease. As mentioned above, ramipril belongs in a class of drugs called angiotensin converting enzyme (ACE) inhibitors which are used for treating high blood pressure and heart failure. In the prevention of cardiovascular disease (CVD), it is often necessary to administer more than one drug, thereby simultaneously reducing a number of CVD risk factors. In the case of the co-administration of one of the statins (simvastatin) and an ACE inhibitor (ramipril), the risk factors associated with LDL cholesterol and blood pressure are treated. As with most drug combination therapies, it is beneficial to administer the actives in one unit dose. LEDDS provides the technology to make this possible.

Simvastatin and ramipril are formulated as separate minicapusles by solubilising/suspending the actives in a suitable medium chain triglyceride (MCT) and forming into seamless microcapsules with an outer gelatin coating according to the methods described in US Pat. Nos. 5, 478,508 and 5,882,680. The formulation of the outer gelatin shell is the same as that outlined in Example 5. The two populations of Simvastatin and ramipril minicapsules, are then blended together and filled into a hard gelatin capsule for oral administration.

### Simvastatin Core Formulation

| Ingredients | % w/w |
|---|---|
| Simvastatin | 10-20 |
| MCT | 80-90 |

### Ramipril Core Formulation

| Ingredients | % w/w |
|---|---|
| Ramipril | 10-20 |
| MCT | 80-90 |

### Example 19a

Sustained release Simvastatin and ramipril minicapsules may also be formulated by coating the seamless minicapsules (described in Example 19), with the rate-controlling polymer coat comprised Eudragit RS and Eudragit RL. The formulation and coating procedure for the Eudragit RL (5% w/w) and Eudragit RS (95% w/w) is the same as that outlined in Example 5a.

### Combinations of the statins with Co-enzyme Q10

### Example 20

Coenzyme Q10 (CoQ 10) or ubiquinone is essentially a vitamin or vitamin-like substance. CoQ10 is known to be highly concentrated in heart muscle cells due to the high energy requirements of this cell type. Congestive heart failure (from a wide variety of causes) has been strongly correlated with significantly low blood and tissue levels of CoQ 10. The severity of heart failure correlates with the severity of CoQ10 deficiency (Proc. Natl. Acad. Sci., 1995, 82, 901-904). As was outlined in Example 19, there are obvious benefits in co-administering drugs in the prevention of cardiovascular disease (CVD). In this case, the co-administration of coenzyme Q10 with simvastatin results in the simultaneous treatment of congestive heart failure and high cholesterol levels respectively. Again LEDDS provides the technology to make this combination product possible.

Simvastatin and coenzyme Q10 are formulated as separate minicapusles by solubilising/suspending the actives in a suitable medium chain triglyceride (MCT) and forming into seamless microcapsules with an outer gelatin coating according to the methods described in US Pat. Nos. 5, 478,508 and 5,882,680. The formulation of the outer gelatin shell is the same as that outlined in Example 5. The two populations of Simvastatin and ramipril minicapsules, are then blended together and filled into a hard gelatin capsule for oral administration. The formulation of the simvastatin is the same as that outlined in Example 19. Both populations of minicapsule can also be coated with a sustained release polymer as described in Example 19a.

### Co-enzyme Q10 Core Formulation

| Ingredients | % w/w |
|---|---|
| Co-enzyme Q10 | 10-20 |
| MCT | 80-90 |

### Combinations of the statins with thiazide diuretics, beta blockers, ACE inhibitors, folic acid, co-enzyme Q10 and anticoagulants

### Example 21

As outlined, the risk of cardiovascular disease can be reduced by treating all the risk factors simultaneously. The risk factors include; LDL cholesterol (treated with simvastatin), blood pressure (treated with ACE inhibitor ramipril, the diuretic hydrochloridethiazide or the calcium channel blocker nimodipine), irregular heart beat (treated with the beta blocker atenolol), serum homocysteine (treated with folic acid), and platelet function (treated with the anticoagulant aspirin). For ease of administration and to simplify the CVD prevention treatment regime, it is preferable that some or all of the drugs mentioned above or formulated into a single dosage form. Obviously with the large number of actives involved, it can be difficult to achieve the drug loadings necessary using conventional dosage forms. The increased solubility conferred on the actives using the LEDDS technology can however be used to achieve the desired loadings.

Simvastatin, coenzyme Q10, ramipril, hydrochlorothiazide, nimodipine, and atenolol minicapusles are prepared by solubilising/suspending the actives in a suitable medium chain triglyceride (MCT) and forming into seamless microcapsules with an outer gelatin coating according to the methods described in US Pat. Nos. 5, 478,508 and 5,882,680. These minicapsules can also be formulated to include required concentrations of aspirin and folic acid either in the core or in the outer gelatin shell. In cases where the drug loadings required are particularly high, extra pharmaceutical active can also be incorporated into the shell. The formulation of the core formulations for the simvastatin, ramipril and co-enzyme Q10 are the same as that Examples 19-20. The different populations of minicapsules (some or all), are then blended together and filled into a hard gelatin capsule for oral administration. The formulation of the simvastatin is the same as that outlined in Example 19. The populations of minicapsule can also be coated with a sustained release polymer as described in Example 19a.

### Hydrochlorothiazide Core Formulation

| Ingredients | % w/w |
|---|---|
| Hydrochlorothiazide | 10-20 |
| MCT | 80-90 |

### Nimodipine Core Formulation

| Ingredients | % w/w |
|---|---|
| Nimodipine | 10-20 |
| MCT | 80-90 |

### Atenolol Core Formulation

| Ingredients | % w/w |
|---|---|
| Atenolol | 10-20 |
| MCT | 80-90 |

### Shell Solution

| Ingredients | % w/w |
|---|---|
| Gelatin | 15-20 |
| Sorbitol/Glycerin | 1-5 |
| Purified Water | 70-80 |
| Folic acid | 1-5 |
| Aspirin | 1-5 |

### ACE inhibitors in combination with cough suppressants

### Example 22

As mentioned previously, angiotensin converting enzyme (ACE) inhibitors are used for treating high blood pressure and heart failure and for preventing kidney failure due to high blood pressure and diabetes. These ACE inhibitors are generally well tolerated, and side effects are usually mild and transient. However one of the more irritating side-effects is a dry, persistent cough has been reported with the use of ramipril and other ACE inhibitors. About 2% to 14% of people who are placed on an ACE inhibitor complain of this chronic cough (Postgrad Med J., 1996, 72, 594-598). Dextromethorphan is one of the most commonly used cough suppressant. Dextromethorphan is preferred as its adverse effects occur in less than 1% of people. By combining dextromethorphan with ramipril, it is possible produce an effective ACE inhibitor without chronic cough side effects.

Ramipril and dextromethorphan are dissolved/suspended into soy bean oil and formed into seamless microcapsules with an outer gelatin shell according to the methods described in US Pat. Nos. 5, 478,508 and 5,882,680. The shell formulation is the same as that outlined in Example 5.

### Core Formulation

| Ingredients | % w/w |
|---|---|
| Ramipril | 5-10 |
| Dextromethorphan | 5-10 |
| Soy bean oil | 80-90 |

### Example 22a

Sustained release ramipril/dextromethorphan minicapsules may also be formulated by coating the seamless minicapsules (described in Example 22), with the rate-controlling polymer coat comprised Eudragit RS and Eudragit RL. The formulation and coating procedure for the Eudragit RL (5% w/w) and Eudragit RS (95% w/w) is the same as that outlined in Example 5a.

### Antimetabolite and aspirin combinations

### Example 23

Hydroxyurea belongs to the group of medicines called antimetabolites. It is used to treat some kinds of cancer and to prevent painful episodes associated with sickle cell anemia. It has been shown that when hydroxyurea is co-administrated with a small dose of aspirin and given to people with thrombocythemia, is more effective in preventing serious bleeding and other complications than anagrelide hydrochloride (the pharmaceutical active currently used to treat the condition) (N Engl J Med., 2005 353, 33-45).

Hydroxyurea (freely soluble in water) decomposes in the presence of moisture and therefore must be formulated in an oil phase. Hydroxyurea and aspirin are solubilised/suspended in a suitable medium chain triglyceride (MCT) and formed into seamless microcapsules with an outer gelatin coating according to the methods described in US Pat. Nos. 5, 478,508 and 5,882,680. The formulation of the outer gelatin shell is the same as that outlined in Example 5.

### Core Formulation

| Ingredients | % w/w |
|---|---|
| Hydroxyurea | 10-20 |
| Aspirin | 1-2 |
| MCT | 80-90 |

### Example 23a

Sustained release hydroxyurea/aspirin minicapsules may also be formulated by coating the seamless minicapsules (described in Example 23), with the rate-controlling polymer coat comprised Eudragit RS and Eudragit RL. The formulation and coating procedure for the Eudragit RL (5% w/w) and Eudragit RS (95% w/w) is the same as that outlined in Example 5a.

### Poorly permeable drugs in combination with permeability enhancers

### Example 24

Small interfering RNA's (siRNA) have emerged as a new and very efficient tool to downregulate gene expression in humans, animals and plants. In particular, high expectations have been given to siRNA as a potential new universal drug for treatment of a variety of human diseases such as cancer, rheumatoid arthritis, brain diseases and viral infections. For instance, it has been shown that the introduction of siRNA targeted against the activated oncogene H-Ras in proliferating cancer cells, is able to revert the cells back into normal cells (Oncogene, 2003, 28, 5694-701) H-Ras is involved in many types of cancer. A major challenge for siRNAs targeting the H-Ras oncogene and all siRNAs is the efficient delivery of siRNA drugs to diseased cells in living animals and eventually in humans. Within the body, naked siRNA is degraded by enzymes. Permeation of the siRNAs across the wall of the intestine is also a common problem.

Thiolated carboxymethylcellulose has been shown to increase the permeability of peptides (Eur J Pharm Biopharm. 2001, 51, 25-32). By combining thiolated carboxymethylcellulose with siRNAs using the LEDDS technology, it may be possible to increase the permeability of siRNAs.

Thiolated carboxymethylcellulose and oncogene targeted siRNA are solubilised/suspended in a suitable medium chain triglyceride (MCT) and formed into seamless microcapsules with an outer gelatin coating according to the methods described in US Pat. Nos. 5, 478,508 and 5,882,680. The formulation of the outer gelatin shell is the same as that outlined in Example 5.

The protease sensitive thiolated carboxymethylcellulose/oncogene targeted siRNA minicapsules are protected from proteolytic attack in the stomach by coating the seamless minicapsules (described above), with the enteric polymer, Eudragit S 12.5, providing zero drug release in the stomach up to 4 hours. The formulation for the Eudragit S 12.5 coating solution is the same as outlined in Example 7a. The coating procedure is the same as that outlined in Example 5a.

### Core Formulation

| Ingredients | % w/w |
|---|---|
| Oncogene targeted siRNA | 5-10 |
| Thiolated carboxymethylcellulose | 5-10 |
| MCT | 80-90 |

### Example 24a

It has long been recognised that nitric oxide (NO) modulates intestinal membrane permeability. A recent study demonstrated that that NO donors may be useful to enhance the intestinal absorption of poorly absorbable drugs (Modulation of intestinal permeability by nitric oxide donors: implications in intestinal delivery of poorly absorbable drugs, Yamamoto A; J Pharmacol Exp Ther. 2001 Jan;296(1):84-90). A number of approaches may be adopted to enhance membrane permeability for poorly soluble drugs, including covalent or non-covalent conjugation of an NO-donor to a poorly permeable drug or co-administering a NO donor with a poorly permeable drug. Ideally, the NO donor should have a very short half-life to ensure a transient increase in membrane permeability. Short half-life NO donors include: Nitroglycerine, L-arginine, Sodium 1-(Pyrrolidin-1-yl)diazen-1-ium-1,2-diolate, Disodium 1-[(2-Carboxylato)pyrrolidin-1-yl]diazen-1-ium-1,2-diolate, Sodium 1-(Piperazin-1-yl)diazen-1-ium-1,2-diolate, Sodium (Z)-1-(N,N-Diethylamino)diazen-1-ium-1,2-diolate. As per poorly permable - polyethylene glycol 3350

### Core Formulation

| Ingredients | % w/w |
|---|---|
| Indinavir | 5-10 |
| Nitroglycerine | 5-10 |
| MCT | 80-90 |

Alternatively the Indinavir and the Nitroglycerine may be formulated as separate minicapusles by again solubilising/suspending in a suitable medium chain triglyceride (MCT) and forming into seamless microcapsules with an outer gelatin coating according to the methods described in US Pat. Nos. 5, 478,508 and 5,882,680. The two populations of saquinavir and cyclosporine A minicapsules, are then blended together and filled into a hard gelatin capsule or sachet for administration.

### Example 24b

The Indinavir / Nitroglycerine minicapsule combination may be gastro-protected by coating the seamless minicapsules (described in Example 7), with the enteric polymer, Eudragit S 12.5, providing zero drug release in the stomach up to 4 hours. The formulation for the Eudragit S 12.5 coating solution is outlined below.

### Enteric Polymer Coating Solution

| Ingredients | % w/w |
|---|---|
| Eudragit S 12.5 | 50 |
| Triethyl citrate | 1-5 |
| Talc | 10-15 |
| Isopropyl alcohol | 40-45 |

### Example 24c

Claudin, an integral component of the inter-cell tight junction apparatus, is vital to maintaining intestinal membrane integrity. Recently, studies demonstrated that a claudin modulator, a segment of C-terminal fragment of *Clostridium perfringens* enterotoxin (C-CPE), dose-dependently enhanced the absorption of dextran (mol. wt. 4000). The effects were not accompanied by injury of the intestinal mucosa as assessed by leakage of lactose dehydrogenase and histological observation. C-CPE was over 400-fold more potent at enhancing dextran absorption than capric acid, a clinically used enhancer of absorption (A Novel Strategy for the Enhancement of Drug Absorption Using a Claudin Modulator, Kondoh et al., Mol Pharmacol 67:749-756,2005)

### Core Formulation 24c

| Ingredients | % w/w |
|---|---|
| Calcitonin | 2-10 |
| Leupeptin | 1-2 |
| Unconjugated deoxycholic acid | 5 |
| C-CPE | 5 |
| Fractionated oat oil | 30 |
| Cremophor EL or TPGS | 25 |
| PEG 400 | 30 |

### Intermediate Solution

| | |
|---|---|
| Linseed oil | 100 |

### Example 24d

### Core Formulation 24d

| Ingredients | % w/w |
|---|---|
| Calcitonin- C-CPE conjugate | 2-10 |
| Leupeptin | 1-2 |
| Unconjugated deoxycholic acid | 5 |
| Fractionated oat oil | 30 |
| Cremophor EL or TPGS | 25 |
| PEG 400 | 30 |

### Intermediate Solution

| | |
|---|---|
| Linseed oil | 100 |

### Peptide, proteolytic enzyme inhibitor and permeability enhancer combination

### Example 25

One of the major problems of delivering biopharmaceuticals (e.g., insulin) via the oral route is their susceptibility to attack by proteolytic enzymes. Although the LEDDS technology allows for biopharmaceuticals to be delivered directly to the small intestine by applying a gastro-protective coating to the minicapsules, it is often beneficial to include a proteolytic enzyme inhibitor in the formulation in order to protect the active from any proteolytic enzymes found in the small intestine. An example of such a proteolytic enzyme inhibitor is leupeptin. Leupeptin inhibits trypsin-like serine proteases such as trypsin, chymotrypsin, chymase, pepsin and thrombin. It also inhibits selected cysteine proteases such as calpain, cathepsin B, H & L and papain. In addition to the incorporation of proteolytic enzyme inhibitors, it is often also necessary to include a permeation enhancer in formulations designed for the intestinal absorption of biopharmaceuticals.

Cyclodextrins can be used as permeability enhancers to deliver actives to the surface of biological membranes. Cyclodextrins are particularly useful when the active is a hydrophobic molecule. It is also known that natural triglycerides can act as permeation enhancers. In this case the intermediate layer of the minicapsule, in addition to functioning as a stability enhancer by preventing any leakage of the core microemulsion, also functions as the permeability enhancer.

An Insulin SEDDS formulation is prepared with polyoxyl hydrogenated castor oil. A formulation consisting of a modified vegetable oil (e.g., polyoxyl hydrogenated castor oil), a surfactant (e.g., TPGS), a co-solvent (e.g., propylene glycol) and a bile salt (e.g., sodium deoxycholate) is prepared by successive addition and mixing of each component. The insulin and leupeptin are then added to the formulation, which is thoroughly mixed to form a clear homogenous mixture. The insulin/leupeptin microemulsion is then formed into seamless microcapsules according to the methods described in US Pat. Nos. 5, 478,508 and 5,882,680 with an intermediate linseed oil layer and an outer gelatin shell. The shell solution formulation is the same as that outlined in Example 5. The insulin/leupeptin minicapsules combination are gastro-protected by coating the seamless minicapsules (described above), with the enteric polymer, Eudragit S 12.5, providing zero drug release in the stomach up to 4 hours. The formulation for the Eudragit S 12.5 coating solution is the same as that outlined in Example 7a. The coating procedure is the same as that outlined in Example 5a.

### Core Formulation

| Ingredients | % w/w |
|---|---|
| Insulin | 2-10 |
| Leupeptin | 1-2 |
| Unconjugated deoxycholic acid | 5 |
| Fractionated oat oil | 30 |
| Cremophor EL or TPGS | 30 |
| PEG 400 | 30 |

### Intermediate Solution

| | |
|---|---|
| Linseed oil | 100 |

### Core Formulation 25a

| Ingredients | % w/w |
|---|---|
| Insulin (PEG-conjugated) | 2-10 |
| Leupeptin | 1-2 |
| Unconjugated deoxycholic acid | 5 |
| Fractionated oat oil | 30 |
| Cremophor EL or TPGS | 30 |
| PEG 400 | 30 |

### Intermediate Solution

| | |
|---|---|
| Linseed oil | 100 |

### Example 26

### Core Formulation

| Ingredients | % w/w |
|---|---|
| Calcitonin | 2-10 |
| Leupeptin | 1-2 |
| Unconjugated deoxycholic acid | 5 |
| Fractionated oat oil | 30 |
| Cremophor EL or TPGS | 30 |
| PEG 400 | 30 |

### Intermediate Solution

| | |
|---|---|
| Linseed oil | 100 |

The gelatin shell formulation, enteric coat and coating procedure are the same as that outlined in Example 25.

### Antigens in combination with adjuvants

### Example 27

Tumor-associated antigens (TAAs) are structures (i.e., proteins, enzymes or carbohydrates) which are present on tumor cells and relatively absent or diminished on normal cells. By virtue of being fairly unique to the tumor cell, TAAs provide targets for the immune system to recognize and cause their destruction. The nature of tumor-associated antigens were unknown for a long time. Many of the initial clinical studies of specific tumor vaccines involved utilizing the tumor cell as a source of TAAs. These tumor cells were obtained from the patient and rendered non-viable by irradiation or killing the tumor cells so that only the membrane fragments remain for use as a vaccine. The tumor cell preparation is then combined with an "adjuvant" prior to administration.

An adjuvant is an agent, such as BCG (Bacillus Calmette-Guerin), which will augment the immune response to TAAs. When considering the oral administration of antigens, stability is major concern. Stability is generally conferred on very labile biomolecules (e.g. antigens) by drying them in sugar glasses. The activity of these biomolecules can however be retained by suspension in perfluorocarbon (PFC) liquid. It is therefore possible to formulate oral antigens in combination with a suitable adjuvant (e.g., BCG, MF59 and Saponin Qs-21) by encapsulating PFC/ glass microsphere biomolecule suspensions in a gelatin capsule (LEDDS) and subsequently coating the capsules to confer protection against proteolytic attack.

Glass microspheres of carcinoembryonic antigen (CEA) (prepared from drying in sugar glasses) are added to PFC liquid and agitated to obtain a homogenous suspension. The carcinoembryonic antigen /PFC suspension is then formed into seamless microcapsules according to the methods described in US Pat. Nos. 5,478,508 and 5,882,680 with an outer gelatin shell. The saponin Qs-21 adjuvant (good aqueous solubility) is added to the gelatin shell solution.

### Core Formulation

| Ingredients | % w/w |
|---|---|
| Carcinoembryonic antigen | 10 |
| PFC Liquid | 90 |

### Shell Formulation

| Ingredients | % w/w |
|---|---|
| Gelatin | 15-20 |
| Sorbitol/Glycerin | 1-5 |
| Saponin Qs-21 adjuvant | 1-10 |
| Purified Water | 70-80 |

### Combination of proton pump inhibitor, anti-H-pylori antibiotic, H-blocker and stomach lining potectant

### Example 28

The proton pump inhibitor omeprazole is formulated in combination with the H-blocker cimetidine as an emulsion core in one population of minicapsules. The oil-in-water emulsion is formed where the both omeprazole (10-20% w/w) is solubilised/suspended in the oil phase (mineral oil) and the cimetidine (10-20% w/w) is solubilised/suspended in the water phase. Both emulsion components are then mixed with the required amount of surfactant (e.g., TPGS). The omeprazole/cimetidine emulsion is then thoroughly mixed to form a clear homogenous mixture. The omeprazole/cimetidine emulsion is then formed into seamless microcapsules according to the methods described in US Pat. Nos. 5, 478,508 and 5,882,680 with an intermediate oil layer and an outer gelatin shell. If necessary, both actives can also be included in the intermediate layer. The formulation for the core emulsion is outlined below. The intermediate and shell layer formulations are the same as that outlined in Example 5. These omeprazole/cimetidine minicapsules are gastro-protected by coating the seamless minicapsules with the enteric polymer, Eudragit S 12.5, providing zero drug release in the stomach up to 4 hours. The formulation for the Eudragit S 12.5 coating solution is the same that outlined in Example 7a outlined below. The coating procedure is the same as that outlined in Example 5a.

A second population of minicapsules is produced containing tetracycline in the core and the stomach lining protectant bismuth subsalicylate in the intermediate layer. By incorporating the bismuth subsalicylate in the intermediate oil layer, the stomach will be protected prior to the release of the antibiotic. The tetracycline core solution and the bismuth subsalicylate intermediate oil phase suspension are then formed into seamless microcapsules according to the methods described in US Pat. Nos. 5, 478,508 and 5,882,680 with an outer gelatin shell. The outer gelatin shell is the same as that outlined in Example 5. The two populations of minicapsules, are then blended together and filled into a hard gelatin capsule for oral administration.

### Minicapsule population 1

### Omeprazole/cimetidine Core Formulation

| Ingredients | % w/w |
|---|---|
| Omeprazole/cimetidine | 10-20 |
| Mineral oil | 10-20 |
| Water | 70-80 |
| TPGS | 1-5 |

### Minicapsule population 2

### Tetracycline Core Formulation

| Ingredients | % w/w |
|---|---|
| Tetracycline | 10-20 |
| PEG 400/water | 80-90 |

### Bismuth subsalicylate Intermediate Solution

| Ingredients | % w/w |
|---|---|
| Bismuth subsalicylate | 10-20 |
| Vegetable oil | 80-90 |

In all cases of the seamless microcapsules technology an active entity may be dispersed in an encapsulating medium, the same or a different active entity may be present in a core, and/or the same or a different active entity may be present in a layer or coating. The active entity may be in a solid or semi-solid form. An active entity solubilised in a solvent or in a liquid phase may alternatively or additionally be present in a core.

There may be a number of different proportions containing the same or different actives in the formulation. Such populations in turn may have sub-populations, for example an active formulation for immediate release, controlled or sustained release with various coatings/layers to control the time and/or location of release of the active. A wide range of possiblities exist within the scope of the invention.

For example, a formulation may comprise a plurality of seamless microcapsules having at least two populations selected from:-
a first minicapsule population in which the minicapsules comprise a core containing an active ingredient and an encapsulating medium, the minicapsules having a diameter of from 0.5 mm to 5 mm;
a second minicapsule population in which the minicapsules comprise a plurality of particles containing an active entity dispersed in an encapsulating medium, the minicapsules having a diameter of from 0.5 mm to 5 mm; and
a third micro or mini particles population in which the minicapsules comprise an inert core and at least one layer around the core, the layer containing an active ingredient.

An example of an active pharmaceutical ingredient used as a model to demonstrate the range of formulations possible is nimodipine. Formulations of a dihydropyrimidine are described in our co-pending PCT application entitled "Dihydropryrimidine Formulations" filed September 27, 2005, the entire contents of which are herein incorporated by reference. Nimodipine is a dihydropyridine derivative and belongs to the class of pharmacological agents known as calcium channel blockers. The contractile processes of smooth muscle cells are dependent upon calcium ions, which enter these cells during depolarisation as slow ionic transmembrane currents. Nimodipine inhibits calcium ion transfer into these cells and thus inhibits contractions of vascular smooth muscle. Nimodipine is a yellow crystalline substance, practically insoluble in water. Nimodipine is typically formulated as soft gelatine capsules for oral administration. Nimodipine is indicated for the improvement of neurological outcome by reducing the incidence and severity of ischemic deficits in patients with subarachnoid haemorrhage from ruptured intracranial berry aneurysms regardless of their post-ictus neurological condition. The precise mode of action is not clear.

### Example 29

| Core Solution | %w/w |
|---|---|
| --Micronised Nimodipine USP/EP | 11.7% |
| --PEG 400 | 46.6% |

| Median Solution | %w/w |
|---|---|
| -- Medium-Chain Triglycerides (MCT) | 2.4% |
| -- Sucrose Acetate Isobutylate (SAIB) | 9.4% |

| Film Solution | %w/w |
|---|---|
| -- Gelatin | 30% |
| -- Purified Water | as required |
| | |
| -- Minicapsule diameter | 1.50-1.80 mm |

The Immediate Release (IR) Nimodipine Multiparticulate Seamless Minicapsules were manufactured according to Freund Industrial Co. Ltd US Patent No. 5,882,680 (Seamless Capsule and Method of Manufacturing Same) and as described in the Summary of the Invention Section. The multiparticulate minicapsules produced in this example achieved an Immediate Release Dissolution Profile as follows.

### Dissolution Method

| | |
|---|---|
| Apparatus: | Vankel VK7025 fully auto mated with Cary Win UV |
| | |
| Dissolution Medium: | Gastric Juice with 1% SDS pH 1.2 (900mls) |
| | |
| Stirring: | USP Apparatus 2 (Paddles) at 100rpm |
| | |
| UV: | 330nm |

| Dissolution Profile of Nimodipine Multiparticulate Immediate Release Seamless Minicapsules Batch MY11 | | | | | | | | | | | | | | | | | |
|---|---|---|---|---|---|---|---|---|---|---|---|---|---|---|---|---|---|
| Time (Mins) | 0 | 15 | 30 | 45 | 60 | 75 | 90 | 105 | 120 | 135 | 150 | 165 | 180 | 195 | 210 | 225 | 240 |
| Batch MY11 % Released | 0 | 8 | 22 | 34 | 44 | 52 | 58 | 64 | 69 | 74 | 78 | 82 | 85 | 89 | 92 | 95 | 99 |

The immediate release product was then filled into hard gelatine capsules to the required dosage strength. Furthermore the invention allows for the immediate release product to be produced in combination with a Sustained Release or Controlled Release multiparticulate minicapsule product in varying ratio's of IR:SR/CR. The immediate release minicapsules can be combined with a Sustained or Controlled release minicapsule component in the following ratio's (w/w by potency) eg. 10% Immediate Release (IR)+90% Sustained (SR)/Controlled Release (CR) minicapsules; 20% IR + 80% SR/CR; 30% IR + 70% SR/CR; 40% IR + 60% SR/CR and 50% IR + 50% SR/CR.

### Example 30

| Core Solution | %w/w |
|---|---|
| -- Micronised Nimodipine USP/EP | 11.7% |
| -- PEG 400 | 46.6% |

| Median Solution | %w/w |
|---|---|
| -- MCT | 2.4% |
| -- SAIB | 9.4% |

| Film Solution | %w/w |
|---|---|
| -- Gelatin | 20.2% |
| -- Sorbitol | 3.0% |
| -- Hydroxypropylmethyl Cellulose Phthlate (HP55) | 6.1% |
| -- Sodium Hydroxide (NaOH) | 0.7% |

The above Example 30 were manufactured according to Freund Industrial Co. Ltd US Patent No.5,882,680 (Seamless Capsule and Method of Manufacturing Same).

In order to control the release (SR) of the Nimodipine over an extended period of time, Hydroxypropylmethyl Cellose Phthalate (HP55) was added to the Film Solution to act as a retarding agent which controlled the release of the Nimodipine over a given period. The multiparticulate minicapsules produced in this example achieved a Sustained/Controlled Release Dissolution Profile as follows.

### Dissolution Method

| | |
|---|---|
| Apparatus: | Vankel VK7025 fully auto mated with Cary Win UV |
| | |
| Dissolution Medium: | Gastric Juice with 1% SDS pH 1.2 (900mls) |
| | |
| Stirring: | USP Apparatus 2 (Paddles) at 100rpm |
| | |
| UV: | 330nm |

| Dissolution Profile of Nimodipine Multiparticulate Sustained Release Seamless Minicapsules Batch MY21 | | | | | | | | | | | | | | | | | |
|---|---|---|---|---|---|---|---|---|---|---|---|---|---|---|---|---|---|
| Time (Mins) | 0 | 15 | 30 | 45 | 60 | 75 | 90 | 105 | 120 | 135 | 150 | 165 | 180 | 195 | 210 | 225 | 240 |
| Batch MY21 % Released | 0 | 2 | 3 | 3 | 4 | 6 | 11 | 21 | 32 | 44 | 55 | 65 | 74 | 82 | 89 | 96 | 101 |

The resultant multiparticulate minicapsules were filled into suitable hard gelatin capsules to the required target strength, typically 30/60/90/120 or 180 mg .Furthermore the invention allows for the combination of the SR/CR multiparticulate minicapsule with an immediate release multiparticulate minicapsule in varying ratio's of SR/CR: IR (%percent Example 30+29). The IR + SR/CR combination ratio's are as per Example 29.

### Example 31

| Core Solution | %w/w |
|---|---|
| -- Micronised Nimodipine USP/EP | 37.5% |
| -- Gelatin | 56.3% |
| -- Sorbitol | 6.3% |
| -- Purified Water | as required |

| Polymer Coating solution | %w/w |
|---|---|
| -- Eudragit RS | 85% w/w |
| -- Eudragit RL | 5% w/w |
| -- Dibutyl Sebacate | 10% w/w |
| -- Talc | as required |
| -- Minicapsule Diameter | 1.50 1.80mm |

The above seamless minicapsules were manufactured in the same way as Example 29 & 30 with the following exceptions:-
1. The core solution was treated with a High Pressure Homogeniser.
2. The median and film solutions were excluded from this example.
3. The polymer coating solution included a 10% plasticiser. The Eudragit RS/RL were adjusted proportionately.

The process used to manufacture the multiparticulate minicapsules in this example in principle was the same as used in Example 29 & 30 with the exception that only a single orifice dosing system was used instead of the normal multiple dosing orifice system. By using a single dosing orifice a uniform solid gelatine pellet or sphere is produced to a specified particle size. This method produces a durable sphere in a gelatine format that includes the active ingredient which in turn allows the sphere or multipaticulate pellet to be further processes with various polymer coating systems. The multiparticulate minicapsules produced in this example achieved a Sustained/Controlled Release Dissolution Profile as follows.

### Dissolution Method

| | |
|---|---|
| Apparatus: | Vankel VK7025 fully auto mated with Cary Win UV |
| | |
| Dissolution Medium: | Gastric Juice with 1% SDS pH 1.2 (900mls) |
| | |
| Stirring: | USP Apparatus 2 (Paddles) at 100rpm |
| | |
| UV: | 330nm |
| | |

| Dissolution Profile of Nimodipine Multiparticulate Sustained Release Seamless Minicapsules Batch MY22 | | | | | | | | | | | | | | | | | |
|---|---|---|---|---|---|---|---|---|---|---|---|---|---|---|---|---|---|
| Time (Mins) | 0 | 15 | 30 | 45 | 60 | 75 | 90 | 105 | 120 | 135 | 150 | 165 | 180 | 195 | 210 | 225 | 240 |
| Batch MY22 % Released | 0 | 2 | 3 | 3 | 4 | 5 | 6 | 8 | 10 | 12 | 15 | 19 | 22 | 26 | 31 | 36 | 41 |

Furthermore the invention allows for the combination of a SR/CR multiparticulate minicapsule with another SR/CR multiparticulate minicapsule and a IR multiparticulate minicapsule or other combinations thereof in varying ratio's of SR/CR:SR/CR:IR (%percent Example 30 + 31 + 29). A population of minicapsules from Example 30, Example 31 and Example 29 in varying ratio's as stated herein below were removed and blended in a suitable mechanical blender.The blended components were then filled into hard gelatine capsule to the required target strength.
Example 30 (45%) + Example 31 (45%) + Example 29 (10%)
Example 30 (50%) + Example 31 (30%) + Example 29 (20%)
Example 30 (30%) + Example 31 (60%) + Example 29 (10%)

### Example 32

| | |
|---|---|
| -- Micronised Nimodipine USP/EP | 500 grams |
| -- Fumaric Acid | 0-125 grams |
| -- Citric Acid | 0-125 grams |
| -- Talc | 5- 250 grams |
| -- Sodium Lauryl Sulphate | 0.125 grams |
| -- Sugar spheres (Non-Pareils) | 250 grams |
| -- Kollidon 30 (Povidone) | 50-150 grams |
| -- Eudragit RL | 5-15 grams |
| -- Eudragit RS | 35- 50 grams |
| -- Isopropyl Alcohol | as required |
| -- Acetone | as required |
| -- Diameter Multiparticulate Spheres | 1.50-1.80 mm |

The above example was produced by the multiparticulate layering process. This drug layering process is a well known and widely used technique in the drug delivery industry and is regularly used by formulation scientist to develop new delivery systems. The Nimodipine Applied Beads (IR) were manufactured as follows.

Nimodipine ,Fumaric Acid or Citric Acid or both, talc and sodium lauryl sulphate (active blend) were blended in a suitable Y-Cone blender. The active blend was applied using a suitable fluid bed system onto non-pareils using a suitable binder or adhering solution, such as Povidone from a suitable organic or aqueous solution such as isopropyl alcohol. The resultant immediate release beads were dried for approx 24 hours. The dried multiparticulate spheres were then screened and the appropriate fractions retained.

The applied beads (IR) were then further processed. A coating solution of a 6.25% solution of Eudragit RS (75-95% w/w) and Eudragit RL (5-25% w/w) dissolved in isopropyl alcohol/acetone mix was sprayed onto the applied beads using a suitable fluid bed system. Talc was added simultaneously via a mechanical feeder to prevent agglomeration. The result was a layered applied sphere with a rate-controlling polymer having a pre-determined dissolution profile.

The resultant coated spheres (SR) from this example were then blended with a percentage of the applied (IR) spheres. The blended spheres from the above were filled into hard gelatine capsules to a target strength.

Furthermore the above example could also be combined with other the examples listed above. The following combinations in varying % percent ratio's can also be produced to give a pre-determined dissolution profile :-
Example 29 + 30 + 31 + 32 or Example 30 + 31 + 32 or Example 31 + 32 and the like. The following ratio's are listed below as examples of the varying combinations that can be produced by removing a partial population of minicapsules from each of the above examples.

Example 29 (10%) + Example 30 (30%) + Example 31 (30%) + Example 32 (30%)
Example 30 (25%) + Example 31 (25%) + Example 32 (50%)
Example 31 (50%) + Example 32 (50%)

### Example 33

| Core Solution | |
|---|---|
| -- Nimodipine USP/EP | 500 grams |
| -- Low Viscosity MCT | 500 grams |

| Film Solution | |
|---|---|
| -- Gelatin | 590 grams |
| -- Sorbitol | 70 grams |
| -- Nimodipine USP/EP | 340 grams |
| -- Purified Water | 2290 grams |

| Polymer Coating Solution | |
|---|---|
| -- Eudragit S | as required |
| -- Isopropyl Alcohol/acetone | as required |
| -- Talc | as required |
| -- Minicapsule Diameter | 1.50-1.80 mm |

The above seamless minicapsules were manufactured in the same way as Example 29 with the following exceptions:-
3. The core solution was pre-treated with an Ultra Centrifugal Mill.
4. The film solution Nimodipine, was pre-treated with a High Pressure Homogeniser
5. The median solution was excluded this formulation.
6. Eudragit S was used as the polymer coat to provide an enteric coat with 0 drug release of up to 4 hours to the minicapsules, to target the drug release to the GIT and providing a pulsed release profile.

A percentage of the Enteric Coated Nimodipine minicapsules and a percentage of the coated minicapsules from Example 29 (as required) and a percentage of the uncoated minicapsules from Example 29 (as required) were blended as per in Example 29 and filled into suitable gelatin capsules to the target strength.

### Example 34

| Core Solution | |
|---|---|
| -- Nifedipine USP/EP | 100-400 grams |
| -- PEG 400 | 400-800 grams |

| Median Solution | |
|---|---|
| -- Low Viscosity MCT | 500-1000 grams |

| Film Solution | |
|---|---|
| -- Gelatin | 590 grams |
| -- Sorbitol | 70 grams |
| -- Nifedipine USP/EP | 100-400 grams |
| -- Purified Water | 1000-2500 grams |

| Polymer Coating Solution | |
|---|---|
| -- Eudragir S | as required |
| -- Isopropyl Alcohol/Acetone | as required |
| -- Talc | as required |
| -- Minicapsule Diameter | 1.50-1.80 mm |

The above seamless minicapsules were manufactured in the same way as Example 29 with the following exceptions: -
1.The Nifedipine core solution was pre-treated with an Ultra Centrifugal Mill.
2.The Nifedipine film solution, was pre-treated with a High Pressure Homogeniser.
3.Eudragit S was used as the polymer coat to provide an enteric coat with 0 drug release of up to 2-4 hours to the minicapsules, to target the drug release to the GIT and providing a pulsed release profile.

### Example 35

| Core Solution | |
|---|---|
| -- Micronised Nifedipine | 500-1000 grams |
| -- Gelatin | 500-3000 grams |
| -- Sorbitol | 0-200 grams |
| -- Purified Water | 4000-6000 grams |

The minicapsules in Example 35 were manufactured according to Examples 29 & 30 and filled into suitable hard gelatin capsules to the required target strength.

### Example 36

1. From Example 34 take a population of Immediate Release (IR) minicapsules.
2. Take a second population of Sustained Release (SR) minicapsules from Example 34
3. In the following ratio 5-25% Immediate Release (IR) and 75-95% Sustained Release (SR) minicapsules calculated by potency from Example 34 are blended using a suitable blender and encapsulated using suitable hard gelatin capsules into the target strengths.

### Example 37

| Core Solution | |
|---|---|
| -- Micronised Nimodipine USP/EP | 100-400 grams |
| -- PEG 400 | 400-800 grams |

| Median Solution | |
|---|---|
| Vegetable Oil or Mineral Oil | 0-1000 grams |

| Mucoadhesive Coating Solution | |
|---|---|
| -- Ethycellulose | 5-100 grams |
| -- PVP | 0.5-50 grams |
| -- Castor Oil | 0-50 grams |
| -- Magnesium Stearate | 0-50 grams |
| -- Acetone | as required |
| -- Isopropanol | as required |

| Film Solution | |
|---|---|
| -- Gelatin | 100-500 grams |
| -- Sumatriptan | 0-100 grams |
| -- Sorbitol | 0-50 grams |
| -- Purified water | 500- 3000 grams |

| Polymer Coating Solution | |
|---|---|
| -- Eudragit RL | 5% w/w |
| -- Eudragit RS | 95% w/w |
| -- Isopropyl Alcohol | as required |
| -- Acetone | as required |
| -- Talc | as required |
| -- Minicapsule Diameter | 0.5-1.80mm |

The Nimodipine Multiparticulate Seamless Minicapsules were manufactured according to freund Industrial Co. Ltd US Patent NO.5,882,680 (Seamless Capsule and Method of Manufacturing Same),as described in the Summary of the Invention Section. This example allows for the inclusion of the active ingredient in the Film Solution (gelatine layer) as also described in the Summary of the Invention Section.

To apply a mucoadhesive coating, a coating solution of 7% ethylcellulose,0.85% PVP and 1% magnesium stearate was dissolved in an isopropanol/acetone mixture. The solution was then sprayed coated onto the minicapsules using a suitable fluidised bed processor. Talc was used to prevent agglomeration of the minicapsules during the spray coating stage. The coated minicapsules were dried in an environmentally controlled drier at 40-50 deg.C for typically 12-24 hours.

In order to further coat the mucoadhesive coated seamless minicapsules, a coating solution of 6.25% Eudragit RL (5%w/w) and 6.25% Eudragit RS (95%w/w) dissolved in isopropyl alcohol/acetone mixture was sprayed coated onto the minicapsules using an automated fluidised bed processor. Talc was used to prevent agglomeration of the minicapsules during the spray coating stage. The coated minicapsules were further dried in an environmentally controlled drier at 40-50deg.C for typically 12-24 hours.

The Nimodipine seamless minicapsules produced in Example 36 were the encapsulated using suitable hard gelatine capsules into typically 30/60/90/120 or 180mg capsules or alternatively formats for rectal, vaginal or nasal administration.

It will be appreciated that any appropriate active ingredients mentioned may be formulated in a similar way to the preceding examples to provide formulations of the invention.

Some embodiments of the present invention will use one or more of the below ingredients in a multiparticulate capsule or sachet. It is noted that the following non-limiting lists illustrate exemplary ingredients that can be used with the present invention, including the broader subclasses and classes to which they belong.
1. *Enzymes* Alpha Galactosidase Amylase Bromelain Cellulase Papain Peptidase Protease Proteolytic Enzymes Superoxide Dismutase Trypsin
2. *Phospholipids* Lecithin Phosphatidyl Choline Phosphatidyl Serine
3. *Specialty Nutraceuticals* 5-Hydroxytryptophan Acetyl L-Carnitine Alpha Lipoic Acid Alpha-Ketoglutarates Bee Products Betaine Hydrochloride Bovine Cartilage Caffeine Cetyl Myristoleate Charcoal Chitosan Choline Chondroitin Sulfate Coenzyme Q10 Collagen Colostrum Creatine Cyanocobalamin (Vitamin B12) DMAE Fumaric Acid Germanium Sesquioxide Glandular Products Glucosamine HCL Glucosamine Sulfate HMB (Hydroxyl Methyl Butyrate) Immunoglobulin (Immune System Support) Lactic Acid L-Carnitine Liver Products Malic Acid Maltose-anhydrous Mannose (d-mannose) MSM Other Carnitine Products Phytosterols Picolinic Acid Pyruvate Red Yeast Extract SAMe Selenium Yeast Shark Cartilage Theobromine Vanadyl Sulfate Velvet Deer Antler Yeast.
4. *Herbal Oils* Aloe Vera Artichoke Oil Artichoke Oil Black Currant Seed Oil 14% GLA Black Currant Seed Oil 15% GLA Borage Oil 20% GLA Borage Oil 22% GLA Boswellia Serrata Oil CLA Conjugated Linolic Acid 75% min. Evening Primrose Oil 10% GLA Evening Primrose Oil 9% GLA Flax Seed Oil 50% ALA Garlic Oil Grape Seed Oil Guggul Lipid Oil Olive Leak Extract Oregano Oil Perilla Oil 60% ALA Pumpkin Seed Oil Pygeum Oil Rosehip Oil Rosemary Oil Saw Palmetto Oil Sterols Tocotrienol Palm Oil Walnut Oil Wheat Germ Oil Sesame Seed Oil Dill Seed Oil Clove Bud Oil Ginger Root Oil Cinnamon Leaf Oil Fennel Seed Oil Curcuma Longa Oil Cummin Seed Oil Celery Seed Oil Coriander Seed Oil Red Rasberry Seed Oil Cranberry Seed Oil Blackberry Seed Oil.
5. *Marine Oils* Cod Liver Oil (1000 A/100 D) Cod Liver Oil (2500A/250D) Fish Oil 30% EPA/20% DHA Fish Oil Concentrated Fish Oil Deodorized Marine Lipid Oil 18/12 Marine Lipid Oil 30/20 Marine Lipid Oil 36/24 Salmon Oil 18% EPA/12% DHA Squalene Oil (Shark)
6. *Other Oils* Alpha Lipoic Acid Cetyl Myristoleate CM Coenzyme Q10. Lecithin Medium Chain Triglycerides MCT.
7. *Vitamins* Ascorbic Acid (Vitamin C) B Vitamins Biotin Fat Soluble Vitamins Folic Acid HCA (Hydroxycitric Acid) Inositol Mineral Ascorbates Mixed Tocopherols Niacin (Vitamin B3) Orotic Acid PABA (Para-Aminobenzoic Acid) Pantothenates Pantothenic Acid (Vitamin B5) Pyridoxine Hydrochloride (Vitamin B6) Riboflavin (Vitamin B2) Synthetic Vitamins Thiamine (Vitamin B1) Tocotrienols Vitamin A Vitamin D Vitamin E Vitamin F Vitamin K Vitamin Oils Vitamin Premixes Vitamin-Mineral Premixes Water Soluble Vitamins
8. *Carotenoids* Apocarotenal Astaxanthin Beta-Carotene Canthaxanthin Carotenoids Lutein/Lutein Esters Lycopene Zeaxanthin
9. *Hormones* 7-Keto-DHEA Androstenedione DHEA Melatonin Nor-Androstenedione Pregnenolone Progesterone 19 Nor-4-Androstendiol 19 Nor-4-Androstenedione 19 Nor-5-Androstenediol 19 Nor-5-Androstendione 3-Indolebutyric Acid 4 Androstendiol 4 Androstendione 6 Furfurylaminopurene 6-Benzylaminopurine
10. *Minerals* Boron Calcium Chelated Minerals Chloride Chromium Coated Minerals Cobalt Copper Dolomite Iodine Iron Magnesium Manganese Mineral Premixes Mineral Products Molybdenum Other Minerals Phosphorus Potassium Selenium Sodium Specialty Minerals Trace Minerals Vanadium Zinc Malic Acid Pyruvate
11. *Probiotics* Acidophilus Bifido Bacteria Lactobacillus, both native wild-type and genetically modified probiotics
12. *Proteins*/*Amino Acids* Amino Acids Betaine Casein Functional Soy Glutamic Acid L-Alanine L-Arginine L-Cysteine L-Glutamine L-Glycine L-Histidine L-Isoeucince L-Leucine L-Lysine L-Methionine L-Ornithine L-Phenylalaline L-Proline L-Taurine L-Threonine L-Tryptophan L-Tyrosine L-Valine N-Acetly-L-Cysteine Protein Soluble Soy Soy Protein Isolates Textured Soy Whey Protein Isolates
13. *Other Embodiments* Specialty Nutrients ATP Forskolin Sterol Esters Stanol EstersProbiotics LactoferinLutein Esters Zeaxanthinlmmunoglobulins Ipriflavone Isoflavones Fructo-Oligo-Saccharides Inulin Huperzine A MelatoninMedicinal MushroomsBile Products Peptone Products Glandular Products Pancreatic Products Thyroid Products Ribose Probiotics Oleo Resins Dill Seed Oleo Resin Black Pepper Oleoresin Capsicum Oleoresin

The present invention can be used in cardiovascular treatments, for example hypertension, heart failure, and heart rhythm disorders. Also, the present invention can be used in immunology (e.g. transplant rejections, auto-immune disorders, etc.). The present invention can be used to treat neurological disorders (such as Parkinson's disease, dementia, stroke, epilepsy, and migraine headache, etc.), psychiatric disorders (schizophrenia, bipolar disease, depression, anxiety, ADHD/ADD, Addictions, etc.), infectious diseases (fungal, bacterial, viral (HIV), etc.), and in anesthesiology (induction anesthesia, local anesthesia). Furthermore, the present invention has application in endocrinology (cholesterol, diabetes, hormone replacement therapy, thyroid dysfunction, oral contraception, obesity, etc.), dermatology (onychomycosis, acne, rosaceae, psoriasis, etc.), rheumatology (arthritis, gout, osteoporosis/Osteomalacia), respiratory fields (asthma, emphysema, cystic fibrosis, etc.), gastro-intestinal fields (gastro-esophageal reflux disease, ulcer prophylaxis, crohn's disease, inflammatory bowel disease, etc.), chronic renal failure (vitamin and mineral replacement, blood pressure regulation, diabetes, depression, etc.), genito-urinary (enlarged prostate/BPH, overactive bladder, erectile dysfunction, feminine yeast infections, etc.) and hematology-oncology (thromboembolous, hermatopoeisis, neoplastic disease, nausea / vomiting).

The present invention further contemplates the use of any active ingredients or medicaments known in the art. The following non-limiting lists illustrate exemplary active ingredients or medicaments and the broader subclasses and classes to which they belong for use in this invention.
1. *Medicaments Acting on the Autonomic Nervous System* (Adrenergic Medicaments, Cholinergic Medicaments,Direct Muscarinic Agonists Choline Esters) acetylcholine bethanechol carbachol methacholine Alkaloidsmuscarine pilocarpine
2. *Direct Nicotinic Agonist* nicotine
3. *Acetylcholinesterase Inhibitors* Acetylcholinesterase Inhibitors ("Reversible") - edrophonium neostigmine physostigmine Acetylcholinesterase Inhibitors ("Irreversible") - diisopropylflurorphosphate (DFP) echothiophate isoflurophate Muscarinic Antagonists Atropine ipratropium pirenzepine copolamine 2-PAM: Acetylcholinesterase Reactivator Pralidoxime (Protopam) {2-PAM}: peripheral acetylcholinesterase reactivator for certain phosphoryl-enzyme complexes
4. *Ganglionic Blockers* hexamethonium mecamylamine trimethaphan atecholamines dobutamine dopamine epinephrine isoproterenol norepinephrine
5. *Direct Adrenoceptor Agonist Medicaments* albuterol clonidine methoxamine oxymetazohne phenylephrine ritodrine salmeterol terbutaline
6. *Indirect-Acting Sympathomimetic Medicaments* amphetamine cocaine ephedrine, Pseudoephedrine tyramine
7. *Alpha-Adrenoceptor Antagonists Medicaments* doxazosin labetalol phenoxybenzamine phentolamine prazosin terazosin tolazoline trimazosin yohimbine
8. *Adrenoceptor Antagonist Medicaments* atenolol butoxaamine esmolol labetalolmetoprolol nadolol pindolol propranolol timolol
9. *Adrenergic Neuron Blocking Medicaments* guanethidine reserpine
10. *Cardiovascular System Disorders Medicaments* (ardiovascular testing and diagnosis Hypertension (HTN) Heart Failure Ischemic Heart Disease Myocardial Infarction Arrhythmias Isolated Diastolic Heart Failure and Cardiomyopathies Cardiac Transplantation Venous Thromboembolism Stroke Hyperlipidemia Peripheral vascular disease) - Diuretics carbonic-anhydrase inhibitors loop diuretics osmotic diuretics potassium sparing diuretics thiazide diuretics Anitiarrhythmic Medicaments Sodium Channel blocking agents isopyramide flecainide ibutilide lidocaine mexiletine moricizine procainamide propafenone quinidine tocainide Calcium Channel blocking agents bepridil diltiazem verapamil Adrenergic receptor antagonists propranolol adenosine amiodarone bretylium disopyramide esmolol sotalol CoA Reductase Inhibitors atorvastatin cerivistatin lovastatin pravastatin simvastatin Bile-acid sequestrants cholestyramine colestipol Fibric acids clofilbrate fenofibrate gemfibrozil niacin, nicotinic acid probucolacebutalol atenolol betaxolol bisoprolol carteolol clonidine labetalcl metoprolol penbutalol pindolol prazosin propranolol timolol Calcium Channel Antagonists amlodipine diltiazem felodipine isradipine nicardipine nifedipine nimodipinenisoldipine verapamil benazepril bepridil captopril enalapril fosinopril lisinopril moexipril quinapril ramipril losartan valasartan amiloride bumetanide chlorothalidone ethacrynic acid furosemide hydrochlorothiazide indapamide metolazone torsemide triamterene
   hydralazine minoxidil nitroprusside prazosin reserpine sotalol spironolactone terazosin Organic nitrates Calcium Channel Antagonists Adrenergic Receptor Antagonists amyl nitrite erythrityl tetranitrate isosorbide dinitrate nitroglycerin
   pentaerythritol tetranitrate phosphodiesterase (PDE) inhibitors anrinone milrinone carvedilol cardiac glycosides digitoxin digoxin diuretics ACE Inhibitors Dobutamine dopamine
11. *Respiratory System Disorders Medicaments* (Asthma Chronic Obstructive Lung Disease (COLD)/Chronic Obstructive Pulmonary Disease (COPD) Acute Respiratory Distress Syndrome (ARDS) Drug-Induced Pulmonary Disease Cystic Fibrosis)Corticosteroids beclomethasone betamethasone cortisone dexamethasone fluticasone (Flovent/Flonase) hydrocortisone methylprednisolone prednisolone prednisone triamcinolone sympathomimetics albuterol salmeterol muscarinic antagonists ipratropium leukotriene pathway inhibitors montelukast zafirtukast mast cell stabilizers cromolyn methylxanthines theophylline aminophylline Dnase
12. Gastrointestinal System Disorders (Gastro-esophageal Reflux Disease (GERD) Peptic Ulcer Disease Inflammatory Bowel Disease Nausea andVomiting Diarrhea, Constipation, Irritable Bowel Disease (IBD) Drug Induced Liver Disease Pancreatitis Viral Hepatitis Liver Transplantation) - Histamine-2 receptor antagonists famotidine nizatidine pantoprazole rabeprazole ranitidineProton Pump Inhibitors esomeprazole lansoprazole omeprazole anticholinergics
   antihistamines (Histamine-i receptor antagonists) dopamine antagonists prokinetic gastric stimulant serotonin 5HT.sub.3 receptor antagonists dolasetron granisetron
   ondansetron hydroxyzine corticosteroids benzodiazepines
   cannabinoids Prokinetic gastric stimulants cisapride
   metoclopramideLaxativesSaline laxatives magnesium salts sodium salts irritant/stimulant medicaments cascara senna phenolphthalein
   bisacodylcasanthranol castor oil methylcellulose psyllium polycarbophil lubricant mineral oil surfactants docusate glycerin lactulose Anti-diarrheal medicaments diphenoxylate atropine diphenoxin loperamide bismuth lactobacillus mesalamine olsalazine
13. *Renal System Disorders Medicaments* (Acute Renal Failure Progressive Renal Failure/Chronic Renal Failure Neurologic System Disorders
   Multiple Sclerosis and inflammatory polyneuropathies Epilepsy Parkinson's disease and Movement Disorders Pain management Headache Amyotrophic Lateral Sclerosis Anti-Epileptic) carbamazepine divalproex sodium felbamate gabapentin lamotrigine oxcarbazepine phenytoin topiramate zonisamide Serotonin 5HT.sub.1d receptor agonists almotriptan frovatriptan naratriptan rizatriptan sumatriptan zolmitriptan ergot alkaloids dihydroergotamine isometheptine/dichlorophenazone caffeine pizotifen benzodiazepines alprazolam clonazepam clorazepate diazepam flumazenil antagonist lorazepam midazolam triazolam barbiturates/Anesthetics pentobarbital phenobarbital thiopental non-depressant anxiolytic buspirone
14. *Treatment of Alcoholism Medicaments* disulfiram
15. *Pain Management Medicaments* Opioids Opioid Peptides beta-endorphin dynorphin enkephalins Agonists codeine etorphine fentanyl hydrocodeine hydromorphone meperidine methadone morphine oxycodone propoxyphene buprenorphine dezocine nalbuphine pentazocine naloxone Non-opiate acetaminophen tramadol
16. *Anti-Parkinsonism Medicaments* levodopa carbidopa bromocriptine pergolide amantadine selegiline anticholinergic agents dopamine Agonists pramipexole ropinirole COMT inhibitors entacapone tolcapone Anti-Spasticity Medicaments baclofen botulinum toxin type A carisoprodol chlorphenesin chlorzoxazone cyclobenzaprine dantrolene diazepam metaxalone methocarbamol orphenadrine tizanidine
17. *Psychiatric System Disorders* (Childhood psychiatric disorders Attention Deficit Hyperactivity Disorder (ADHD)/Attention Deficit Disorder (ADD) Eating disorders Alzheimer's disease and Dementia Disorders Substance abuse and Addictive Disorders alcohol, tobacco and caffeine abuse Schizophrenia Depressive disorders Bipolar disorders Anxiety disorders Obsessive-Compulsive disorders Sleep disorders)Psychostimulant medicaments amphetamine mixed salts dextroamphetamine methylphenidate Antipsychotic Medicaments Phenothiazine type chlorpromazine fluphenazine Thioxanthene type thiothixene Butyrophenone type haloperidol Dibenzodiazepine type clozapine Thienobenzodiazepine type olanzapine quetiapine Antidepressant Medicaments Tricyclic antidepressants amitriptyline clomipramine also a SSRI desipramine doxepin imipramine maprotiline nortriptytine protriptyline Monoamine oxidase inhibitors (MAO-I's) clorgyline (specific for MAO type A) isocarboxazid phenelzine tranylcypromine Second Generation Medicaments (not including SSRIs) amoxapine bupropion netazodone trazodone Serotonin-Specific Reuptake Inhibitors (SSRIs) citalopram clomipramine escitalopram fluoxetine fluvoxamine paroxetine sertraline lithium mirtazapine venlafaxine
18. *Anti-Anxiety Agents* barbiturates benzodiazepines buspirone chloral hydrate doxepin hydroxyzine sedative-hypnotics serotonin reuptake inhibitors
19. *Anti-Demential Medicaments* cholinesterase inhibitors donepezil galantamine rivastigmine tacrine
20. *Endoctimologic System Disorders Medicaments* (Diabetes mellitus Thyroid disorders Adrenal Gland disorders Pituitary Gland disorders ACTH Adrenal androgens Adrenocortical Function Antagonists Mineralocorticoid antagonists)-Anti-Diabetic Medicaments Insulin Sulfonylureas acetohexamide chlorpropaamide glimepiride glipizide glyburide tolazarnide tolbutamide Biguanides metformnin Alpha-glucosidase Inhibitors acarbose miglitol
   Thiazolidinedione Derivatives pioglitazone rosiglitazonetroglitazone Thyroid Disorder Medicaments Levothyroxine Liothyronine Liotrix Hypothalamic and Pituitary Gland Medicaments bromocriptine chorionic gonadotropin (hCG) corticotropin generic (ACTH) cosyntropin desmopressin gonadorelin acetate (GnRH) gonadorelin hydrochloride (GnRH) goserelin acetate growth hormone histrelin leuprolide menotropins (hMG) natarelin octreotide oxytocin pergolide protirelin sermorelin (GHRH) somatrem somatropin thyrotropin (TSH) urofollitropin vasopressin
21. *Gynecologic System and Obstetric Conditions Medicaments* (Pregnancy and Lactation Infertility Contraception Menstruation-related disorders Endometriosis Hormone Replacement Therapy (HRT) )Conjugated estrogens desogestrel di-norgestrel ethinyl diacetate ethinyl estradiol levonorgestrel medroxyprogesterone norethindrone norgestimate progesterone
22. *Urologic System Disorders Medicaments* (Erectile Dysfunction Benign Prostatic Hypertrophy Urinary Incontinence)apomorphine alprostadit phosphodiesterase (PDE-5) inhibitors sildenafil tadalafil vardenafil tolterodine tamulosin yohimbine
23. *Immunologic System Disorders Medicaments* (Systemic Lupus Erythematosus and other Collagen-vascular diseases Allergic and pseudo-allergic drug reactions Bone and Joint System Disorders Osteoporosis and Osteomalacia Rheumatoid Arthritis Osteoarthritis Gout and hyperuricemia) - Medicaments used in the Control of Inflammation Non-steroidal anti-inflammatory drugs (NSAIDs) aspirin diclofenac diflusnisal etodolac fenoprofen flubiprofen ibuprofen indomethacin ketoprofen ketorolac meclofenamate nabumetone naproxen oxaprozin phenylbutazone piroxicam salicytate sulindac tolmetin
   Cyclocxygenase-2 inhibitors (COX-2) celecoxib rofecoxib Arthritis and Gout Medicaments allopurinol chloroquine colchicine enbrel Glucocorticoids Gold methotrexate NSAIDs Penicillamine alendronate raloxifene
24. *Disorders of the Eyes, Ears, Nose, and Throat Systems Medicaments* (Glaucoma Allergic rhinitis) Histamine-1 receptor antagonists brompheniramine cetirizine chlorpheniramine clemastine cyproheptadine dimenhydrinate diphenhydramine doxylamine fexofenadine loratidine Sympathomimetic medicaments pseudoephedrine
25. *Dermatologic System Disorders Medicaments* (Acne Psoriasis Rosacea and pigmentation disorders Hematologic System Disorders Hematopoeisis Anemias Coagulation disorders Sickle-cell anemia Drug-induced hematologic disorders)
26. *Coagulation Disorders Medicaments -* aspirin clopidogrel fibrinolytic inhibitors fibrinolytics glycoprotein (GP) IIb/IIIa antagonists/monoclonal antibodies abciximab eptifibatide tiofibran heparin low-molecular weight heparins Plasma fractions-blood factors ticlopidine vitamin K warfarin
*27. Vaccines, toxoids, and other immunobiologics*
28. *Antibiotics* Penicillins amoxicillin ampicillin benzathine Penicillin G benzyl Penicillin carbenicillin cloxacillin dicloxacillin methicillin mezlocillin nafcillin oxacillin phenoxymethyl Penicillin piperacillin ticarcillin Cephalosporins 1 st generation: cefazolin cephalexin cephatothin 2nd generation: cefaclor (Ceclor) cefoxitin (Mefoxin) cefpodoxime (Vantin) cefuroxime (Zinacef, Ceftin) loracarbef (Lorabid) 3rd generation: cefoperazone cefotaxime (Claforan) cefotetan ceftazidime (Fortax, Taxidime, Tazicef) ceftriaxone (Rocephin) veftizoxime (Cefizox) 4th generation: cefepime Other beta-Lactams aztreonam (Azactan) clavulanic acid imipenem (Primaxin) meropenem (Merrem IV) sulbactam Other Cell-Wall Synthesis Inhibitors bacitracin cycloserine fosfomycin vancomycin
29. *Agents Which Affect Cell Membranes* Polymixins Colistimethate Potymyxin B
30. *Protein Synthesis Inhibitors* Aminoglycosides amikacin gentamicin kanamycin neomycin netilmicin streptomycin tobramycin Tetracyclines demeclocycline doxycycline doxycyclrnue tetracycline Macrolides azithromycin clarithromycin erythromycin esters erythromycin Other Protein Synthesis Inhibitors Chloramphenicol (Chloromycetin) Clindamycin (Cleocin) Spectinomycin (Trobicin) Inhibitors of Folate-Dependent Pathways co-trimoxazole silver Sulfadiazine sodium Sulfacetamide sulfamethoxazole (Gantanol) sulfasalazine (Azulfidine) (Salicylazosulfapyridine) sulfisoxazole (Gantrisin) sulfonarnides Dihydrofolate Reductase Inhibitor trimethoprim
31. *DNA Gyrase Inhibitors* ciprofloxacin gatifloxacin levofloxacin lomefloxacin nalidixic acid ofloxacin
32. *Urinary Tract Antiseptics* nitrolurantoin
33. *Antimyobacterial Agents* First-line anti-TB medicaments ethambutol isoniazid (INI-I) pyrazinamide rifampin (Rimactane) streptomycin Second-line anti-TB medicaments capreomycinA cycloserine dapsone ethionamide para-aminosalicylic acid
34. *AntiFungal Agents* amphotericin B clotrimazole fluconazole flucytosine griseofulvin itraconazole ketoconazole miconazole nystatin
35. *AntiParasitic Agents* Antimalarials chloroquine mefloquine primaquine pyrimethamine-sulfadoxine Anti protozoals metronidazole pentamidine isethionate pyrimethamine-sulfonamide trimethoprim sulfamethoxazole
36. *Antihelminthic Medicamentsmebendazole* praziquantel pyrantel pamoate thiabendazole
37. *Antiviral Medicaments* acyclovir didanosine foscarnet ganciclovir ribavirin rimantadine stavudine valacyclovir vidarabine zalcitabine zidovudine
38. *Protease inhibitors* indinavir ritonavir saquinavir
39. *Oncologic and Immunological Disorders Medicaments* (Breast Cancer Lung Cancer Colorectal Cancer Prostate Cancer Malignant Lymphomas Ovarian Cancer Acute Leukemias Chronic Leukemias Melanoma and other Skin Cancers Hematopoeitic Stem Cell Transplantation) - Anti-Neoplastic Medicaments Alkylating Agents busulfan carboplatin carmustine cisplatin cyclophosphamide ifofamide lomustine mechlorethamine meiphalan procarbazine thiotepa Antimetabolites folic acid Antagonist methotrexate Purine Antagonists 6-mercaptopurine 6-thioguanine Pyrimidine Antagonists cytarabine fluorouracil Hormonal Agents: Hormones diethylstilbestrol estrogens prednisone Modulation of Hormone Release & Action Aminoglutethimide leuprolide acetate tamoxifen Plant Alkaloids Vinca Alkaloids vinblastine vincristine Podophyllotoxins Etoposide (VP-16) Others- docetaxel paclitaxel Antibiotics - bleomycin dactinomycin daunorubicin doxorubicin mitomycin Other Anti-neoplastic Medicaments amsacrine azathioprine capecitabine chlorambucil cyclosporine 5 gemcitabine hydroxyurea mitotane mitoxantrone pamidronate
40. *Immunosuppressant Medicaments* 15-desoxyspergualin corticosteroids cyclosporine Interferons Interleukins mycophenolate mofetil sirolimus (rapamycin) tacrolimus thalidomide
41. *Nutritional Disorders Medicaments* (Malnutrition, vitamin and mineral deficiencies Enteral Nutrition Obesity) orlistat appetite suppressants sympathomimetic stimulants amphetamine stimulants Mineral supplementation calcium ion iodine iron magnesium ion phosphorous potassium ion selenium sodium ion zinc Fat-soluble vitamins vitamin A vitamin D vitamin E vitamin K Water-soluble vitamins vitamin C thiamine (vitamin B1) riboflavin (vitamin B2) niacin (vitamin B3) pyridoxine (vitamin B6) folate cyanocobalamin (vitamin B12)
42. *Medicaments used to Alleviate Symptoms of Allergic Rhinitis Upper Respiratory Symptoms, Cough, Mild Aches and Pains* Nasal Decongestants ephedrine phenylephrine phenylpropanolamine pseudoephedrine Antihistamines (Histamine-1 receptor antagonists)
43. *Antitussive agents* benzonatate codeine dextromethorphan Expectorants guaifenesin iodinated glycerol terpin hydrate Xanthines aminophylline caffeine dyphylline theophylline Pain relievers narcotic agonists NSAIDS acetam inophen
44. *Dietary Supplements* Arnica Bilberry Black Cohosh Cat's claw Chamomile Echinacea Evening Primrose Oil Fenugreek Flaxseed Feverfew Garlic Ginger root Ginkobiloba Goldenrod Hawthorn Kava-Kava Licorice Milk thistle Psyllium Rauwolfia Senna Soybean St. John's wort Saw palmetto Turmeric Valerian
45. *Therapeutic Proteins and Biotechnology Medicaments*
46. *Additional Agents* Norvasc, Neurontin, Paxil, Augmentin, Propecia, Lamisil, Lescol, bisphosphonate. abacavir sulfate acetazolamide acetylsalicylic acid albendazole allopurinol amiloride hydrochloride amitriptyline hydrochloride artemether atropine sulfate benznidazole biperiden hydrochloride chloroquine phosphate chlorpheniramine maleate chlorpromazine hydrochloride cimetidine ciprofloxacin hydrochloride clofazimine clomiphene citrate clomipramine hydrochloride cloxacillin sodium codeine phosphate dapsone didanosine diethylcarbamazine citrate digoxin diloxanide furoate DL-methionine Doxycycline Efavirenz ergometrine maleate ergotamine tartrate erythromycin ethyl succinate ethambutol hydrochloride ethosuximide ferrous sulfate alendronate sodium amlodipine besylate amphetamineatorvastatin calcium benazepril hydrochloride bisoprolol fumarate bupropion hydrochloride carbidopa cefprozil cetirizine hydrochloride citalopram hydrobromide clindamycin hydrochloride clonidine hydrochloride clopidogrel bisulfate cyclobenzaprine hydrochloride desloratadine digoxin diltiazem hydrochloride doxazosin mesylate doxycycline enalapril maleate fexofenadine hydrochloride fluoxetine hydrochloride folic acid fosinopril sodium hydrocodone bitartrate hydrocodone hydroxyzine hydrochloride indinavir irbesartan isosorbide mononitrate lamivudine levothyroxine sodium lopinavir loratadine losartan potassium meclizine hydrochloride medroxyprogesterone acetate meperidine metformin hydrochloride methylphenidate hydrochloride methylprednisolone metoclopramide hydrochloride) minocycline hydrochloride montelukast sodium naproxen sodium nelfinavir nevirapine niclosamide nicotinamide nifurtimox nitrofurantoin nortriptyline hydrochloride oxybutynin chloride oxycodone hydrochloride paracetamol paroxetine hydrochloride penicillin V potassium phenytoin sodium pioglitazone hydrochloride prednisolone primaquine phosphate pravastatin sodium prednisolone promethazine hydrochloride promethazine fumarate propylthiouracil pyrantel embonate pyridostigmine bromide raloxifene hydrochloride ranitidine hydrochloride rifampicin risedronate sodium risperidone rosiglitazone maleate salbutamol sulfate saquinavir mesylate sertraline hydrochloride sildenafil citrate sulfadiazine sumatriptan succinate tamoxifen citrate tamsulosin hydrochloride temazepam terazosin hydrochloride timolol maleate tolterodine tartrate tramadol hydrochloride trazodone hydrochloride triclabendazole valacyclovir hydrochloride valdecoxib valproic acid valsartan venlafaxine hydrochloride verapamil hydrochloride warfarin sodium zolpidem tartrate
47. Nucleic acids, single and double stranded DNA, modified DNA, RNA, mRNA, SiRNA, gene vectors, antisense oligonucleotides, decoy oligonucleotides, ribozymes, single and double stranded aptamers.

Various delivery systems may be used for any of the active ingredients. Such delivery systems are described in our co-pending PCT application filed September 27, 2005, and entitled "Minicapsule Formulations", the entire contents of which are herein incorporated by reference.

The invention is not limited to the embodiments hereinbefore described which may be varied in detail.

## Claims

1. A formulation comprising a plurality of seamless multiparticulate minicapsules having a diameter of from 0.5 mm to 5 mm, the minicapsules having an encapsulating medium, and the mincapsules containing at least two different active ingredients.

2. A formulation as claimed in claim 1 wherein the two active ingredients exhibit incompatibility such as oil versus water soluble, long versus short half-life, differential circadian-related illness drug chronotherapy requirements.

3. A formulation as claimed in claim 1 in which the active ingredients have differential inherent drug solubility, permeability and stability profiles.

4. A formulation as claimed in claim 1 in which one active ingredient is of low solubility, low permeability or unstable with short half-life.

5. A formulation as claimed in any of claims 1 to 4 in the form of a single unit being a capsule, tablet, pellet or pill.

6. A formulation as claimed in any of claims 1 to 5 wherein the individual drugs are separated to avoid direct contact.

7. A formulation as claimed in any of claims 1 to 6 wherein at least some of the minicapsules comprise a core.

8. A formulation as claimed in claim 6 wherein the core of some of the minicapsules is solid or semi-solid at room temperature.

9. A formulation as claimed in any of claims 1 to 8 wherein at least some of the minicapsules comprise a shell.

10. A formulation as claimed in claims 1 to 7 wherein at least some of the minicapsules are beads.

11. A formulation as claimed in any of claims 1 to 7 wherein at least some of the minicapsules are beads made from a mixed suspension of shell/core materials.

12. A formulation as claimed in claim 7 wherein at least one of the active entities in the core is in solid or semi-solid form or solubilized in a solvent or in a liquid phase.

13. A formulation comprising a plurality of seamless multiparticulate beads having a diameter of from 0.5 mm to 5 mm, the beads having an encapsulating medium, and the beads containing at least two different active ingredients.

14. A formulation as claimed in claim 10, 11 or 13 wherein at least one of the active entities in the bead is in solid or semi-solid form or solubilized in a solvent or in a liquid phase.

15. A formulation as claimed in claim 1 wherein one of the active ingredients is subject to one or more of the following: (a) poor drug absorption from the intestine; (b) drug metabolism by enzymes of the cytochrome P450 class; (c) drug degradation by low pH in the stomach; (d) drug degradation by enzymes in the small intestine; (e) efflux from cells by cellular pump systems; (f) development of resistance following ongoing drug administration; (g) targets an individual molecular pathway of a disease stemming from multiple molecular pathway dysfunction; and wherein another of the active ingredients is (i) a metabolism inhibitor, including cytochrome P450 inhibitors; (ii) a degradative enzyme inhibitors; (iii) an efflux inhibitor; or (iv) targets a different individual molecular pathway of a disease stemming from multiple molecular pathway dysfunction.

16. A formulation as claimed in any of claims 1 to 15 comprising at least two populations of sustained release minicapsules having different in-vitro dissolution profiles.

17. A formulation as claimed in any of claims 1 to 16 wherein at least one active ingredient is in powder form and at least another active ingredient is in liquid/emulsion form.

18. A formulation as claimed in any of claims 1 to 16 wherein the encapsulating medium of at least some of the minicapsules contains at least one of the active ingredients.

19. A formulation as claimed in any of claims 7 to 17 wherein the core contains at least one active ingredient.

20. A formulation as claimed in any of claims 1 to 18 wherein at least some of the minicapsules comprise at least one layer.

21. A formulation as claimed in claim 19 wherein the layer contains at least one active ingredient.

22. A formulation as claimed in claim 18 or 19 wherein the layer comprises a coating to control the time and/or location of the release of the active entity.

23. A formulation as claimed in claims 21 wherein at least one coating is an immediate release coating.

24. A formulation as claimed in any of claims 22 or 23 wherein at least one coating is a sustained release coating.

25. A formulation as claimed in any of claims 21 to 23 wherein the coating comprises a sustained release and an immediate release coating.

26. A formulation as claimed in any of claims 21 to 24 wherein at least one coating is an enteric coating.

27. A formulation as claimed in any of claims 21 to 25 wherein at least one coating is a bioadhesive coating.

28. A formulation as claimed in claim 26 wherein the bioadhesive coating is a mucoadhesive coating.

29. A formulation as claimed in any of claims 1 to 27 wherein the-encapsulating solution contains an active ingredient.

30. A formulation as claimed in any of claims 1 to 28 wherein at least some of the minicapsules are formed from a solution containing the encapsulating medium and an active ingredient.

31. A formulation as claimed in any of claims 1 to 29 comprising at least two different populations of minicapsules.

32. A formulation as claimed in claim 30 wherein one population of minicapsules contains a first active ingredient and another population of minicapsules contains a second active ingredient.

33. A formulation as claimed in any of claims 1 to 32 which is:-
• a combination product comprising a methylxanthine and an anticancer agent (such as cisplatin, paclitaxel, daubomycin or vincristine);
• a combination product comprising a methylxanthine and a Vitamin A analogue (such as valproaic acid, valproate or isotretinoin);
• a combination product comprising a methylxanthine and a nitric oxide donor such as nitroprusside, 02-acyl diazenium diolole or NO-NSAIDs such as NO-aspirin;
• a combination product comprising a methylxanthine and a reactive oxygen species scavenger such as stephenhenanthrine or uvariopsine;
• a combination product comprising an immunostimatory agent such as inosine or other adjuvants and an anticancer agent such as cisplatin, paclitaxel, daubomycin or vincristine;
• a combination product comprising various antiretroviral agents for the treatment of HIV/AIDS, selected from sequinivir, stavudine, ritonivir, lipinavir, amprenevir;
• a combination product comprising various antiretroviral agents for the treatment of HIV/AIDS together with immunostimulatory agents;
• a combination product for the treatment of malaria comprising Artemisinin-based actives, including artesunate plus sulfadoxine/pyrimethamine or artesunate and amodiaquine;
• a combination product for the treatment of tuberculosis comprising isoniazid, rifampin and pyrazinamide;
• a combination product for the co-treatment of HIV/AIDS, Malaria and TB, comprised of, from one of the following: HIV: Sequinivir, Stavudine, Ritonivir, Lipinavir, or Amprenevir; Malaria: Sulfadoxine/Primethamine/Artesunate; and Tuberculosis: Isoniazid/Rifampin/Pyrazinamide;.
• a combination product comprising various cardiovascular agents, selected from one or more of ACE inhibitors, antidiuretics, statins, anticholesterol agents, anti-coagulants, beta-blockers and anti-oxidants;
• a combination product comprising immunomodulators including vaccines and immunotherapeutic agents with immunostimulatory and/or adjuvants;
• a combination product comprising a proton pump inhibitor (PPI) [which may be selected from omeprazole, lansoprazole, rabeprazole, esomeprazole, pantoprazole], an anti-H-Pylori antibiotic [which may be selected from metronidazole, tetracycline, clarithromycin, amoxicillin], H-blockers [which may be selected from cimetidine, ranitidine, famotidine, nizatidine] and stomach lining protectants [such as bismuth subsalicylate], the PPI and H-blockers being released following transit through the stomach, the antibiotic release in the stomach and the stomach lining protectant being released in the stomach;
• a combination product comprising agents susceptible to efflux pump activity or metabolism via cytochrome P450 subtypes, including 3A, together with inhibitors of such;
• a combination product comprising an antibiotic susceptible to enzymatic degradation and a degradative enzyme, the antibiotic have a controlled release profile in the stomach and small intestine and the enzyme being released in the distal small intestine and colon;
• a combination product comprising a narcotic, anti-psychotic or other potentially addictive agent with an antidote or irritant, the former drug classes being released in the stomach and small intestine with the antidote, an innocuous or non-systemically absorbed agent, being released in the colon, the irritant being irritating when injected but innocuous when taken orally;
• a combination product for the treatment of Alzheimer's Disease comprising a cholinesterase inhibitor (such as donepezil, rivastigmine, galantamine) and a N-Methyl-D-Aspartame (NMDA) antagonist such as memantine;
• a combination product for the treatment of Alzheimer's Disease comprising a cholinesterase inhibitor (such as donepezil, rivastigmine, galantamine) and one or more from the following classes: vitamins, statins, estrogen, nootrophic agents, ginkgo biloba, anti-inflammatory agents, anti-depressants, anti-psychotics, mood stabilizers and calcium channel blockers, including Nimodipine;
• a cholesterol lowering combination product comprised of a HMG-CoA inhibitor and a intestinal cholesterol uptake inhibitor;
• a combination product for the treatment of diabetes comprising insulin and an insulin sensitizer;
• a combination product for the treatment of diabetes comprising insulin and an oral antihyperglycemic agent;
• a combination product for the treatment of diabetes comprising insulin and a sulfonylurea agent or metformin;
• a combination product for the treatment of diabetes comprising insulin and an oral PTP-1B inhibitor;
• a combination product for the treatment of diabetes comprise an oral memetic agent with an appetite suppressant or fat uptake inhibitor such as orlistat;
• a combination product comprising an anti-cancer agents and a potency enhancers, including isoflavanoids, polyphenols and anti-cancer agent derivatives;
• a combination product containing a potency enhancer such as an isoflavanoid and either a heart disease therapy, osteoporosis therapy, autoimmune disease treatment or inflammatory bowel disease treatment.

34. A pharmaceutical composition comprising a capsule containing the formulation of any of claims 1 to 33
